(19) **Europäisches Patentamt European Patent Office Office européen des brevets**

(11) **EP 4 291 217 B1**

(12) **FASCICULE DE BREVET EUROPEEN**

(45) Date de publication et mention de la délivrance du brevet:
**01.04.2026 Bulletin 2026/14**

(21) Numéro de dépôt: **22702744.8**

(22) Date de dépôt: **09.02.2022**

(51) Classification Internationale des Brevets (IPC):
**A61K 36/28** *(2006.01)* **A61P 31/14** *(2006.01)*

(52) Classification Coopérative des Brevets (CPC):
**A61P 31/14; A61K 36/28; C12Y 103/98001;**
A61K 2236/00; Y02A 50/30

(86) Numéro de dépôt international:
**PCT/EP2022/053144**

(87) Numéro de publication internationale:
**WO 2022/171682 (18.08.2022 Gazette 2022/33)**

(54) **COMPOSITION THÉRAPEUTIQUE À BASE DE FEUILLES DE NEUROLAENA INHIBITRICE DE LA DHODH POUR LE TRAITEMENT D'INFECTIONS PAR VIRUS À ARN**

**THERAPEUTISCHE ZUSAMMENSETZUNG BASIEREND AUF NEUROLAENA BLÄTTER ZUR HEMMUNG DER DHODH UND BEHANDLUNG VON RNA VIRUS INFEKTIONEN**

**THERAPEUTIC COMPOSITION BASED ON LEAVES OF NEUROLAENA FOR INHIBITING DHODH AND TREATING ARN VIRUS INFECTIONS**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.02.2021 FR 2101262**
**05.01.2022 FR 2200066**

(43) Date de publication de la demande:
**20.12.2023 Bulletin 2023/51**

(73) Titulaire: **PHYTOBOKAZ**
**97113 Gourbeyre (FR)**

(72) Inventeur: **JOSEPH, Henry**
**97113 Gourbeyre (FR)**

(74) Mandataire: **Rhein, Alain**
**Cabinet BREV & SUD**
**55 Avenue Clément Ader**
**34170 Castelnau-le-Lez (FR)**

(56) Documents cités:
**EP-A2- 1 169 036** **FR-A1- 2 870 457**
**US-B1- 6 841 561**

- **NICOLON C ET AL: "Insuffisance rénale aiguë chez un enfant au cours d'une infection par le virus de la dengue", ARCHIVES DE PEDIATRIE, ELSEVIER, PARIS, FR, vol. 23, no. 1, 10 November 2015 (2015-11-10), pages 53 - 55, XP029371086, ISSN: 0929-693X, DOI: 10.1016/ J.ARCPED.2015.09.021**
- **BEDOYA L M ET AL: "Guatemalan plants extracts as virucides against HIV-1 infection", PHYTOMEDICINE, ELSEVIER, AMSTERDAM, NL, vol. 15, no. 6-7, 20 June 2008 (2008-06-20), pages 520 - 524, XP022711350, ISSN: 0944-7113, [retrieved on 20080220], DOI: 10.1016/ J.PHYMED.2007.10.006**
- **GRACIOSO JULIANO S ET AL: "Antinociceptive Effect in Mice of a Hydroalcoholic Extract of Neurolaena lobata (L.) R. Br. and its Organic Fractions", JOURNAL OF PHARMACY AND PHARMACOLOGY : JPP, vol. 50, no. 12, 12 April 2011 (2011-04-12), GB, pages 1425 - 1429, XP055917744, ISSN: 0022-3573, DOI: 10.1111/ j.2042-7158.1998.tb03370.x**

EP 4 291 217 B1

- NAYAK BIJOORSHIVANANDA ET AL: "Neurolaena lobata L. promotes wound healing in Sprague Dawley rats", INTERNATIONAL JOURNAL OF APPLIED AND BASIC MEDICAL RESEARCH, vol. 4, no. 2, 1 January 2014 (2014-01-01), pages 106 - 110, XP055917756, ISSN: 2229-516X, DOI: 10.4103/2229-516X.136791
- ANONYMOUS: "Sciences: Le laboratoire guadeloupéen Phytobokaz démontre l'efficacité de l'herbe à pique sur les virus émergents à ARN comme la Covid | Outremers360", 11 February 2021 (2021-02-11), XP055835871, Retrieved from the Internet <URL:https://www.outremers360. com/bassin-atlantique-appli/ sciences-le-laboratoire-guadeloupeen-phytobo kaz-demontre-lefficacite-de-lherbe-a-pique-sur- les-virus-emergents-a-arn-comme-la-covid> [retrieved on 20210830]

**Description**

Les virus à génome ARN sont responsables de nombreuses pathologies humaines telles que par exemple la grippe, la dengue, l'hépatite C, la rougeole, la bronchiolite des nourrissons ou encore, plus récemment, le coronavirus dit Covid-19.

L'arsenal thérapeutique classique de lutte contre les virus à génome ARN consiste à cibler l'activité d'une protéine virale essentielle au cycle du virus. De telles protéines essentielles peuvent notamment consister en l'ARN polymérase, l'intégrase, une hélicase ou une protéase. Cependant, bien que ces protéines virales puissent être conservées chez plusieurs virus à génome à ARN, le développement de molécules thérapeutiques à large spectre, actives contre différents virus, reste relativement limité.

De plus, la plasticité des génomes viraux, ainsi que la capacité d'adaptation et d'évolution des virus à génome ARN, facilitent l'apparition rapide de mutants d'échappement. Ces mutants rendent inefficaces le traitement par les molécules thérapeutiques à large spectre qui ciblent une protéine virale essentielle au cycle du virus.

Afin de contourner ces problèmes de mutation des cibles thérapeutiques, une nouvelle approche thérapeutique ciblant la cellule hôte, plutôt que le virus lui-même, a été développée ces dernières années. Le principe de cette nouvelle approche est de bloquer les mécanismes cellulaires essentiels à la réplication virale, de sorte à empêcher la prolifération virale en stimulant la réponse immunitaire innée dans les cellules infectées par un virus à génome ARN.

L'un des mécanismes cellulaires connu, essentiel à la réplication virale, est le cycle de biosynthèse de la pyrimidine. En effet, la pyrimidine est cruciale pour la survie des cellules humaines, notamment lorsque ces dernières sont des cellules hôtes de pathogène, notamment des virus à génome ARN.

Plus particulièrement, chez les mammifères et dans les cellules humaines, la synthèse de la pyrimidine se fait par deux voies de biosynthèses : la « voie de novo pyrimidine », et, une autre « voie de sauvetage » qui est réalisée dans des conditions physiologiques particulières. La plupart des parasites humains ne possèdent pas la « voie de sauvetage » de synthèse des pyrimidines. Or, les pyrimidines sont nécessaires à la fabrication des nucléotides pyrimidiques. Ces nucléotides pyrimidiques sont essentiels à la survie de la cellule et à sa multiplication. Ainsi, le blocage de la « voie de novo pyrimidine » est considéré comme un moyen thérapeutique efficace pour cibler sélectivement les parasites humains sans affecter l'hôte humain et son fonctionnement cellulaire normal.

En d'autres termes, tous les pathogènes humains, dont les virus à génome ARN, sont déficitaires de la « voie de sauvetage » de biosynthèse de la pyrimidine. Par conséquent, la « voie de novo pyrimidine », de la cellule hôte, est une voie ciblée en thérapie pour éliminer ces pathogènes, notamment pour empêcher la réplication des virus à ARN dans les cellules hôtes.

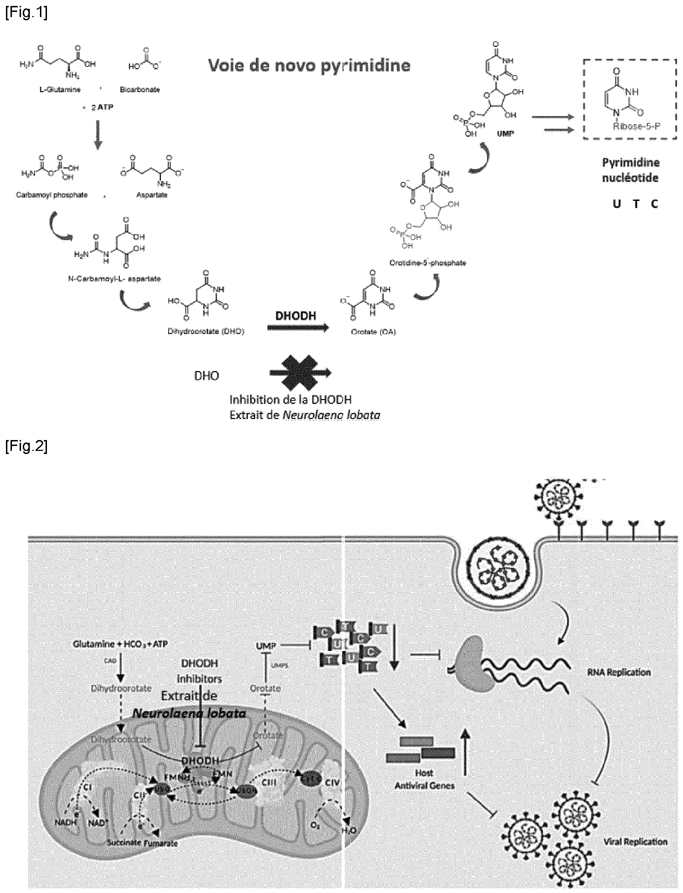

La voie de biosynthèse de novo pyrimidine est une voie de synthèse qui se fait en plusieurs étapes successives. Cette voie et ses différentes étapes sont illustrées sur la figure 1 des dessins ci-joints.

Plus particulièrement, la quatrième étape de la voie de biosynthèse de novo pyrimidine consiste en une déshydrogénation du dihydroorotate dit « DHO » qui conduit à la formation d'orotate « ORO ».

L'enzyme, de type oxydoréductase, qui catalyse la réaction de déshydrogénation du dihydroorotate, est la dihydroorotate déshydrogénase dite « DHODH ».

Lors de la déshydrogénation du DHO en ORO, il y a un transfert d'électron entre deux cofacteurs, dont l'un est donneur d'électrons et l'autre est accepteur d'électrons. Par exemple, le transfert d'électron, lors de cette réaction de déshydrogénation du dihydroorotate, se fait grâce au couple d'oxydoréduction des flavines mononucléotides $FMN/FMNH_2$ avec le couple des ubiquinones $QH_2/Q$ ou avec le couple nicotinamide adénine NAD+/NADH. La DHODH se lie à son cofacteur FMN en conjonction avec l'ubiquinone pour catalyser l'oxydation du dihydroorotate en orotate.

La voie de biosynthèse de novo des pyrimidines assure la synthèse d'uridine 5-monophosphate dite UMP. L'UMP sert de précurseur pour les autres nucléotides pyrimidiques. Ces derniers sont nécessaires et indispensables à la division cellulaire et à l'activité métabolique de la cellule hôte qui est infectée par le virus à génome ARN. Ainsi, il a été démontré qu'inhiber la DHODH des cellules hôtes entraine une chute de la quantité de pyrimidines dans les cellules infectées ce qui amplifie la réponse immune innée antivirale.

De manière connue, les enzymes DHODH sont séparées en deux groupes, les DHODH de classes 1 et les DHODH de classe 2. Ces deux classes de DHODH sont établies en fonction de leur similarité de séquence, de leurs sites de fixation, de leur localisation cellulaire et de leur substrat préféré.

Les DHODH classe 1 sont des enzymes cytosoliques présentes chez les pathogènes de type protozoaires.

Les DHODH classe 2 sont des enzymes de type protéine monomérique se liant à la membrane interne des mitochondries des eucaryotes.

En d'autres termes, chez l'homme, la DHODH, appartient à la classe 2, c'est une protéine mitochondriale située sur la surface externe de la membrane mitochondriale interne. La DHODH humaine présente deux domaines constitués par le domaine alpha / bêta-baril contenant le site actif et le domaine alpha-hélicoïdal, ce dernier forme l'ouverture d'un tunnel menant au site actif.

A ce jour, il existe plusieurs inhibiteurs connus de la DHODH humaine, tels que le brequinar, le tériflunomide ou encore le léflunomide. EP1169036 et US6841561 divulguent l'utilisation d'inhibiteurs de la DHODH (Leflunomide et Brequinar) pour traiter des infections à virus ARN.

Par exemple, le léflunomide est utilisé pour traiter la polyarthrite rhumatoïde ou la sclérose en plaques. Les effets immunosuppresseurs du léflunomide ont été attribués à l'épuisement de l'apport de pyrimidine pour les cellules T ou encore à des voies plus complexes médiées par l'interféron ou l'interleukine. L'inhibition de la DHODH humaine, par le léflunomide est due à la fixation du léflunomide sur le domaine alpha-hélicoïdal qui forme l'ouverture d'un tunnel menant au site actif de la DHODH. Bien que commercialisé en tant que principe actif de médicament, le léflunomide entraine des effets secondaires chez 1% à 10% des patients, notamment de type diarrhées, nausées, vomissements, aphtes, douleurs abdominales, inflammation du côlon, maux de tête, inflammation des tendons, accentuation de la chute naturelle des cheveux, eczéma, peau sèche, augmentation des transaminases, ou encore baisse des globules blancs.

L'inhibition de la DHODH humaine par le brequinar s'effectue par le même mécanisme que le léflunomide, en occupant l'ouverture du tunnel menant au site actif. Le brequinar été utilisé comme anticancéreux à la fin des années 1980 ; néanmoins comme il est responsable de multiples effets secondaires indésirables, il n'a pas été accepté comme médicament. De plus, le brequinar est reconnu comme très toxique pour les cellules humaines. En conséquence, l'idée de l'utiliser en thérapie pour lutter contre des infections virales a été très vite abandonnée par le corps médical.

Ainsi, dans le cas du développement d'une pathologie liée à une infection virale par un virus à génome à ARN, les inhibiteurs connus de la DHODH semblent inappropriés pour traiter les symptômes viraux d'un patient, en particulier du fait de leurs effets secondaires et cytotoxiques sur les cellules, sans distinction du type de cellule.

C'est la raison pour laquelle, dans le cas d'une infection virale, notamment par un virus à ARN, il convient de trouver une solution alternative aux inhibiteurs connus de la DHODH, tels que le brequinar, le tériflunomide ou encore le léflunomide. Cette solution alternative, en plus d'empêcher la voie de novo pyrimidine par inhibition de l'activité enzymatique de la DHODH, est souhaitée non cytotoxique pour l'ensemble des cellules humaines et avec un minimum d'effets secondaires en cas de prise sous forme de médicament.

De plus, dans le contexte actuel, la plupart des patients est attachée à l'origine et aux modes de conception des médicaments. En général, les patients souhaitent minimiser au maximum la part des composants synthétiques constituant le médicament. Ces composants synthétiques peuvent être responsables des effets secondaires tout comme le principe actif. En conséquence, en plus d'inhiber la DHODH humaine, l'objectif de la présente invention consiste également à trouver un inhibiteur de la DHODH qui soit autant que possible d'origine naturelle, de conception simple avec une faible empreinte carbone, afin de limiter les effets secondaires tous en traitant l'infection au virus à ARN et les symptômes associés.

L'invention est exposée dans le jeu de revendications joint. La présente invention a pour but de pallier les inconvénients de l'état de la technique en proposant une composition thérapeutique comprenant une teinture mère d'un extrait de feuilles d'une plante appartenant au genre « *Neurolaena* » et à l'espèce « *lobata* » pour utilisation en tant que médicament inhibiteur de la dihydroorotate déshydrogénase humaine (DHODH).

Cette composition thérapeutique a pour avantage d'avoir, en tant que principe actif inhibiteur de l'activité de la DHODH, ladite teinture mère de *Neurolaena lobata*. Cette teinture mère est avantageusement facile et rapide à préparer, à moindre couts à échelle industrielle. De plus, ladite teinture mère de la composition thérapeutique de l'invention est avantageusement d'origine naturelle, c'est à dire non synthétique, tout en étant reconnue dans la littérature comme étant non cytotoxique *in vivo.*

En conséquence, ladite composition thérapeutique présente peu, voire pas du tout, d'effets secondaires liés à son principe actif d'origine naturelle et non cytotoxique. Suite à l'ingestion par un patient, ladite composition thérapeutique de l'invention inhibe l'activité de la DHODH sans avoir d'effet cytotoxique. La prise de ladite composition thérapeutique, incluant ladite teinture mère, génère une augmentation de la réponse immune innée chez des patients dont le système immun est affaibli par des pathogènes ou toutes autres maladies.

De plus, selon d'autres caractéristiques de l'invention, ladite composition thérapeutique est pour une utilisation en tant que médicament dans le traitement de symptômes associés à une infection virale de virus à génome à ARN.

Ladite composition thérapeutique, comprenant une teinture mère de *Neurolaena lobata* ayant pour propriété d'inhiber l'activité de la DHODH et d'empêcher la voie de novo de synthèse de la pyrimidine, est appropriée dans le cas d'infection virale par un virus à ARN, notamment à ARN positif.

En effet, les virus à ARN sont déficitaires de la « voie de sauvetage des pyrimidines ». Les virus à ARN pour leur mécanisme d'infection cellulaire doivent utiliser la « voie de novo » de la cellule hôte pour synthétiser la pyrimidine qui est nécessaire dans le mécanisme de réplication cellulaire, autrement dit nécessaire à la multiplication virale. Ainsi, l'inhibition de la voie de novo de la cellule hôte empêche la réplication cellulaire, c'est-à-dire la réplication virale des virus à ARN. C'est pourquoi l'utilisation de ladite composition thérapeutique de l'invention en tant que médicament permettant de traiter les symptômes associés à une infection virale de virus à génome à ARN est appropriée.

Selon un mode de réalisation préféré, la composition thérapeutique de l'invention s'utilise en tant que médicament dans le traitement des symptômes associés à une infection virale de virus à génome à ARN choisi parmi les familles de virus de la liste suivante : les Coronaviridae, les Flaviviridae, les Orthomyxoviridae, ou encore les Togaviridae.

Tout préférentiellement, la présente invention est également relative à un procédé de préparation d'un extrait sec d'une teinture mère diluée de feuilles sèches de *Neurolaena lobata,* caractérisé en ce qu'il comporte les étapes suivantes :

i) on prépare un mélange à base de feuilles sèches de *Neurolaena lobata* dans de l'alcool de canne à sucre à 50°, ledit mélange présentant une concentration massique comprise entre 15 et 20 g/L ;

ii) on laisse ledit mélange à macérer sous agitation pendant environ 21 jours ;

iii) Après macération, on filtre le mélange sur « cartouches » de porosité 50-75 μm et on obtient un filtrat liquide, correspondant à une teinture mère de feuilles sèches de *Neurolaena lobata,* et un rétentat solide ;

iv) on dilue ladite teinture mère liquide à ¼ par ajout d'une solution aqueuse et on obtient alors une solution hydroalcoolique ;

v) on effectue une évaporation de l'alcool contenu dans cette solution par évaporateur rotatif, jusqu'à obtention d'une solution aqueuse ;

vi) on congèle la solution aqueuse ainsi obtenue, puis on la lyophilise pour obtenir un extrait sec de la teinture mère diluée de feuilles sèches de *Neurolaena lobata.*

De manière avantageuse, le mélange préparé à l'étape i) présente une concentration massique comprise entre 16 et 17g de feuilles sèches de *Neurolaena lobata* par litre (L) d'alcool de canne à sucre à 50°, et de préférence égale à 16g/L.

Tout préférentiellement, lors de l'étape iv), on dilue ladite teinture mère en mélangeant un volume égal à 0,75L de filtrat et un volume égal à 2,25L d'eau.

L'invention est également relative à un procédé de préparation d'une solution liquide à partir d'un extrait sec de teinture mère diluée et lyophilisé de feuilles sèches de *Neurolaena lobata,* ledit extrait sec étant obtenu suivant le procédé décrit précédemment, ladite solution liquide étant obtenue en diluant ledit extrait sec dans de l'eau ou dans un solvant aqueux pharmaceutiquement acceptable, ladite solution liquide présentant une concentration comprise entre 6 500 et 20 000 ng d'extrait sec par mL de solvant aqueux, de préférence entre 6 667 et 20 000 ng/mL.

Dans la présente demande, on entend par solvant pharmaceutiquement acceptable un solvant pouvant être utilisé dans la préparation d'une composition pharmaceutique et qui présente les caractéristiques d'être non toxique et biologiquement acceptable pour une utilisation vétérinaire de même que pharmaceutique humaine.

La présente demande concerne également un extrait de teinture mère de feuilles sèches de *Neurolaena lobata* diluée, selon une dilution au ¼ de la teinture mère, et lyophilisé, ledit extrait étant en solution et présentant une concentration finale comprise entre 6 500 et 20 000 ng/mL (masse d'extrait sec lyophilisé de teinture mère diluée / volume de solution aqueuse), pour son utilisation dans le traitement d'une infection virale due au virus SARS-CoV-2 responsable de la Covid 19.

Ledit extrait de teinture mère de feuilles sèches de *Neurolaena lobata* diluée tel que décrit ci-dessus est susceptible d'être obtenu selon le procédé décrit précédemment.

L'extrait de teinture mère de feuilles de *Neurolaena lobata* est tout particulièrement indiqué pour une utilisation en tant que médicament dans la diminution de la production de cytokines, notamment IL-6 et IP-10, dans le traitement des formes graves d'une infection virale due au virus SARS-CoV-2 responsable de la Covid 19.

D'autres caractéristiques et avantages de l'invention ressortiront de la description détaillée qui va suivre des modes de réalisation non limitatifs de l'invention, en référence aux figures annexées, dans lesquelles :

[FIG.1] : la figure 1 représente schématiquement les étapes de la voie de synthèse de novo pyrimidine dans les cellules humaines, dont la quatrième étape qui consiste en la réaction de déshydrogénation, c'est à dire l'action de l'enzyme dihydroorotate deshydrogénase « DHODH » pour transformer le dihydroorotate « DHO » en orotate ORO ;

[FIG 2] : la figure 2 représente schématiquement l'action inhibitrice de ladite composition de l'invention sur la DHODH humaine mitochondriale, au sein d'une cellule hôte humaine infectée par un virus à génome ARN ;

[FIG.3] : la figure 3 montre la courbe d'évolution, en fonction du temps de contact entre la DHODH et son substrat, de la densité optique mesurée à 600 nm d'un échantillon à une concentration de 0,01 μg/mL constitué soit par du brequinar, soit par une teinture mère d'un extrait de feuilles de *Neurolaena lobata,* nommé « H1 », ou soit encore par une autre teinture mère d'un extrait de feuilles de *Neurolaena lobata* nommé « H2 » ;

[FIG.4] : la figure 4 montre la courbe d'évolution, en fonction du temps de contact entre la DHODH et son substrat, de la densité optique mesurée à 600 nm d'un échantillon à une concentration de 0,1 μg/mL constitué soit par du brequinar, soit par « H1 », ou soit encore par « H2 » ;

[FIG.5] : la figure 5 montre la courbe d'évolution, en fonction du temps de contact entre la DHODH et son substrat, de la densité optique mesurée à 600 nm d'un échantillon à une concentration de 1 μg/mL constitué soit par du brequinar, soit par « H1 », ou soit encore par « H2 » ;

[FIG.6] : la figure 6 représente la courbe d'évolution du pourcentage d'inhibition de l'activité de la DHODH sur son substrat, observée entre 0 et 275 secondes de mise en présence avec l'échantillon, en fonction de la concentration de l'échantillon H1 ou H2 ;
[FIG.7] : la figure 7 représente la courbe d'évolution du pourcentage d'inhibition de l'activité de la DHODH sur son substrat, observée entre 0 et 275 secondes de mise en présence avec l'échantillon, en fonction de la concentration de l'échantillon H1, H2 ou de brequinar.

La présente invention décrit une composition thérapeutique comprenant une teinture mère d'un extrait de feuilles d'une plante appartenant au genre « *Neurolaena* » et à l'espèce « *lobata* » pour une utilisation en tant que médicament inhibiteur de la dihydroorotate déshydrogénase humaine (DHODH).

La figure 1 représente schématiquement la voie de novo de synthèse des pyrimidines comprenant plusieurs étapes, dans laquelle l'étape 4 implique l'action de la dihydroorotate déshydrogénase humaine « DHODH ».

Dans la voie de novo de synthèse des pyrimidines, les précurseurs du noyau pyrimidique sont la glutamine, l'acide aspartique et le $CO_2$. Tel que visible sur la figure 1, dans la première étape, il y a formation du carbamylphosphate par la carbamylphosphate synthétase. En seconde étape, l'aspartate transcarbamylase catalyse la formation du carbamylaspartate. Ce dernier est transformé à la troisième étape, par la dihydroorotase, en dihydroorotate « DHO ». Puis en quatrième étape, la dihydroorotate déshydrogénase « DHODH » va catalyser la transformation du « DHO » en orotate « ORO » qui servira de précurseur réactionnel pour obtenir l'Uridine monophosphate « UMP ». L'UMP est utile dans le mécanisme de polymérisation avec l'ARN nécessaire à la multiplication cellulaire.

Selon l'invention, la teinture mère d'un extrait de feuilles d'une plante appartenant au genre « *Neurolaena* » et à l'espèce « *lobata* » est avantageusement d'origine naturelle. En effet, *Neurolaena lobata* est une plante appartenant à la famille des Astéracées que l'on retrouve dans les Antilles et en Amérique centrale, notamment en Guadeloupe. Sur ces territoires, cette plante se cultive et se récolte facilement car les conditions agroécologiques et pédoclimatiques y sont favorables.

Ainsi, par son origine naturelle, l'usage d'un extrait de *Neurolaena lobata* permet avantageusement de diminuer le risque d'effets secondaires lors de son utilisation au sein d'une composition thérapeutique pour traiter une maladie.

En outre, pour conforter cette idée, plusieurs études et publications scientifiques ont démontré qu'un extrait de *Neurolaena lobata* ne présente aucune toxicité *in vivo* (Gracioso J.S. et al., J. Pharm. Pharmacol. 1998, 50 : 1425 - 1429 ; Gracioso J.S. et al., Phytomedecine, 2000, Vol. 7(4), pp.283 - 289). En particulier, chez des souris, après ingestion, par voie orale, d'une dose de 5000 mg/kg d'extrait hydroalcoolique des parties aériennes de *Neurolaena lobata,* aucune toxicité physiologique n'a été observée après plusieurs jours.

En conséquence, en plus de son origine naturelle, le choix d'un extrait de *Neurolaena lobata,* en tant que principe actif d'une composition thérapeutique visant à inhiber l'activité de la DHODH, respecte avantageusement l'objectif de l'invention d'être non cytotoxique pour l'ensemble des cellules humaines, en particulier celles impliquées dans la réponse immune, aux concentrations qui ont été choisies.

Selon l'invention, ladite teinture mère d'un extrait de feuilles de *Neurolaena lobata* consiste en une solution hydroalcoolique.

Selon un mode de réalisation privilégié, la teinture mère est réalisée à partir d'un extrait uniquement de feuilles d'une plante de *Neurolaena lobata.*

Selon l'invention, ladite composition thérapeutique est sous une forme galénique pour une ingestion par voie orale. Par exemple, ladite composition thérapeutique est sous une forme liquide, par exemple sous la forme d'un sirop, ou encore sous une forme solide, par exemple sous la forme d'un comprimé.

Selon l'invention, ladite composition thérapeutique comprend en tant que principe actif ladite teinture mère de *Neurolaena lobata* possédant un effet inhibiteur de l'activité de la DHODH, ainsi que d'autres excipients permettant sa formulation galénique. Les interactions pouvant exister entre ces excipients et ladite teinture mère n'influencent et n'impactent pas l'effet inhibiteur de la DHODH.

De préférence, ladite composition thérapeutique de l'invention comprend uniquement des excipients d'origine naturelle, qui n'engendrent pas ou peu d'effets secondaires lorsqu'ils sont en formulation avec ladite teinture mère de *Neurolaena lobata.*

Selon un mode de réalisation préféré, ladite composition thérapeutique est utilisée en tant que médicament dans le traitement des symptômes associés à une infection virale.

Plus spécifiquement, ladite composition thérapeutique de l'invention est utilisée en tant que médicament dans le traitement des symptômes associés à une infection par un virus à génome à ARN choisi parmi les familles de virus de la liste suivantes : les Coronaviridae, les Flaviviridae, ou encore les Togaviridae :

- parmi les Coronaviridae, on citera à titre d'exemple le SARS-CoV-2, responsable de la Covid 19 ;
- parmi les Flaviviridae, on citera le genre Hepacivirus dont le seul représentant est le virus responsable de l'hépatite C, et, dans le genre Flavivirus, le virus responsable de la fièvre jaune ou encore le virus Zika et,
- parmi les Togaviridae, on citera le virus responsable du chikungunya.

En effet, pour leur réplication virale au sein de la cellule hôte, les virus à ARN ont besoin que la voie de novo de synthèse des pyrimidines de la cellule hôte soit fonctionnelle. Ces virus sont dépourvus d'une voie de synthèse des pyrimidines. Cependant, si cette voie cellulaire est bloquée par l'usage de la composition thérapeutique de l'invention inhibant l'activité de la DHODH, la réplication virale des virus à ARN au sein de la cellule hôte ne sera plus possible, ces derniers étant déficitaires à la fois de la voie de novo mais aussi de la voie de sauvetage de synthèse de la pyrimidine.

La figure 2 montre le mécanisme d'action de la composition de l'invention sur l'inhibition de la DHODH dans une cellule humaine infectée par un virus à génome à ARN. Grâce à la teinture mère d'un extrait de feuilles de *Neurolaena lobata* de la composition de l'invention, la réaction de déshydrogénation réalisée par la DHODH dans la mitochondrie n'a pas lieu. La voie de novo de synthèse de la pyrimidine est bloquée, et la voie de sauvetage n'est pas utilisable par le virus qui en est dépourvu. L'UMP et les bases pyrimidiques des nucléotides ne peuvent pas être utilisées par le virus à ARN pour démarrer la réplication virale.

En conséquence, aucun virion ne peut être fabriqué par la cellule hôte, même après introduction du génome viral à ARN au sein de ladite cellule hôte. L'inhibition de la DHODH empêche la multiplication virale des virus à génome ARN et empêche la production et la sortie de virions à l'extérieur de la membrane cellulaire de l'hôte. Ainsi, l'usage de la composition de l'invention, à action inhibitrice de l'activité de la DHODH empêche la multiplication virale et le développement des symptômes associés à la présence du pathogène au sein de l'organisme. La composition de l'invention constitue donc un moyen thérapeutique efficace pour traiter les infections virales, notamment par des virus à génomes ARN.

Il en résulte que l'utilisation de la composition thérapeutique de l'invention en tant que médicament dans le traitement des symptômes associés à une infection par un virus à génome ARN consiste en une solution pour lutter contre l'infection et limiter la multiplication virale.

La composition thérapeutique de l'invention est une bonne alternative aux solutions existantes de traitement de maladie virale ciblant l'inhibition de la DHODH. Cette utilisation spécifique permet d'empêcher la réplication virale au sein de la cellule hôte, en inhibant la voie de novo de synthèse de la pyrimidine, tout en augmentant la réponse immune par les cellules de défense et sans effet cytotoxique.

La présente composition thérapeutique de l'invention consiste donc en une solution alternative permettant d'inhiber la DHODH, c'est-à-dire de bloquer la voie de novo de synthèse des pyrimidines nécessaire à la réplication des virus à ARN, ceci sans être invasive et destructrice des cellules impliquées dans la réponse immune contre le pathogène.

La composition thérapeutique de l'invention a également pour avantage d'être facile à fabriquer tout en étant le plus naturelle possible aux yeux des consommateurs et des patients qui en feraient l'usage.

Les résultats d'expériences ci-dessous sont destinés à illustrer l'effet d'inhibition de la DHODH par la composition thérapeutique de l'invention.

D'autres résultats d'expériences obtenus *in vitro* détaillés ci-dessous illustrent, notamment, l'action de la composition thérapeutique de l'invention en particulier sur les formes graves causées par une infection virale au virus du SARS-CoV-2 responsable de la Covid-19.

En effet, ces résultats démontrent une action particulièrement intéressante de la composition thérapeutique de l'invention comprenant une teinture mère d'un extrait de feuilles d'une plante de *Neurolaena lobata* sur la diminution du taux de certaines cytokines libérées par les cellules après une infection par le virus du SARS-CoV-2.

Les cytokines sont des protéines naturellement synthétisées par les cellules immunitaires pour médier la réponse immunitaire suite à une infection par un pathogène. Elles favorisent une réaction inflammatoire naturelle permettant à l'organisme infecté de se défendre contre le pathogène.

Or, dans certains cas d'infections par le SARS-CoV-2, la libération de cytokines, notamment dans les cellules pulmonaires est si importante qu'elle déclenche un « orage cytokinique ». Cet emballement du système immunitaire débouche sur une réaction hyper-inflammatoire susceptible de détruire les tissus, de provoquer des syndromes de détresse respiratoire aiguë, pouvant entraîner des détériorations physiologiques, voire de devenir létale pour la personne chez laquelle cette réaction se déclenche.

Les résultats de tests, illustrés ci-après, menés *in vitro* sur une composition thérapeutique conforme à l'invention comprenant une teinture mère d'un extrait de feuilles d'une plante de *Neurolaena lobata* démontrent que la production de cytokines peut être substantiellement diminuée par l'action de ladite composition.

Pour en revenir à présent aux résultats de tests relatifs à l'inhibition de l'action de la DHODH, des tests ont été menés sur une composition thérapeutique de l'invention comprenant une teinture mère d'un extrait de feuilles d'une plante de *Neurolaena lobata.*

Cette teinture mère constitue l'échantillon à tester. La mesure de l'inhibition de l'action de la DHODH sur son substrat est réalisée dans une plaque multi puits classique à paroi transparente. Les puits contiennent les échantillons à tester, la DHODH et son substrat.

Pour évaluer l'inhibition de la DHODH par l'échantillon, on utilise le paramètre de la densité optique dite « DO ». En effet, on mesure, dans chaque puit, la « DO » à une longueur d'onde de 600 nm, à plusieurs intervalles de temps, pendant une durée de 5 min.

Plus spécifiquement, chaque puit comprend l'échantillon dilué ou non, l'enzyme de la DHODH et son substrat colorimétrique dilué dans un tampon test. Ledit substrat colorimétrique comprend de la DHO qui peut être transformée en ORO par action de la DHODH.

Au cours du temps, la consommation du substrat colorimétrique DHO par l'enzyme DHODH se traduit par une diminution de la DO. Cette diminution signifiant la transformation de la DHO colorée en ORO non coloré par l'activité de la DHODH. En d'autres termes, la DHO est consommée, réduite en ORO par l'activité de la DHODH, ce qui provoque une modification de la DO mesurée.

En cas d'inhibition de l'activité de la DHODH par l'échantillon, la DO reste stable au cours du temps. En effet, dans le cas d'une inhibition, le substrat DHO colorimétrique ne sera pas transformé par la DHODH en ORO, la DO restera donc celle de la DHO initiale.

Pour mener les expériences, il a été utilisé :

- une solution d'enzyme de classe 1 nommée « rhDHODH » pour « recombinant humain DHODH »,
- un tampon test constitué de 50 mM Tris, 150 mM KCI et 0,1% Triton ® X-100, à pH 8,
- un mélange de substrat de la DHODH contenant : 2 mM de L-dihydroorotique dit « DHO », 0,2 mM de décylubiquinone dit « Q » et 0,12 mM de 2,6 Dichloroindophenol sodium salt hydrate dit « DPIP » dans le tampon test,
- un échantillon de teinture mère d'un extrait de feuilles d'une plante de *Neurolaena lobata,* dilué ou non, dans un tampon de dilution constitué de diméthylsulfoxyde dit « DMSO ».

Il est à noter que dans le mélange de substrat de la DHODH, le L-dihydroorotique dit « DHO » constitue le substrat colorimétrique utilisé par la DHODH lors de la réaction de déshydrogénation.

Dans le mélange de substrat, la décylubiquinone dit « Q » et le Dichloroindophenol sodium salt hydrate dit « DPIP » sont les accepteurs et donneurs d'électrons. Le transfert de ces électrons permet la réalisation de la réaction de déshydrogénation par l'oxydoréductase DHODH.

<u>Préparation de la teinture mère d'un extrait de feuilles d'une plante de *Neurolaena lobata* constituant l'échantillon H1 :</u>

La teinture mère H1 d'un extrait de feuilles d'une plante de *Neurolaena lobata* est obtenue en réalisant les étapes de procédé suivantes :

- On récolte les feuilles de *Neurolaena lobata,*
- On sèche lesdites feuilles,
- On broie lesdites feuilles sèches jusqu'à obtention d'une poudre,
- On fait macérer la poudre dans une solution d'alcool de canne à sucre pendant 21 jours jusqu'à obtention d'un macérat, le ratio des quantités étant 1 g de poudre pour 62,5 mL de solution d'alcool de canne à sucre,
- On filtre ledit macérat jusqu'à obtention d'un filtrat, nommé H1.

Dans le procédé susmentionné, selon un mode de réalisation préféré, on peut sécher les feuilles sous un courant d'air chaud, à une température de préférence inférieure à 40°C, pendant environ 120 heures, jusqu'à ce qu'elles présentent un taux d'humidité résiduelle de l'ordre de 6 à 9, de préférence de 6,5 à 9%.

Le taux d'humidité est déterminé par toutes méthodes appropriées connues de l'homme du métier. Par exemple, le taux d'humidité peut être déterminé en utilisant un dessiccateur installé dans une pièce présentant une température inférieure à 40°C, avec un taux d'humidité relative inférieure à 85% sans exposition directe aux rayons du soleil, au courant d'air ou aux vibrations.

Par exemple, le dessiccateur référencé XM60 commercialisé par PRECISA MOLEN France, avec une précision standard de 1 mg en haute résolution et des plages de température allant de 30°C à 230°C avec une incrémentation de 1°C peut être utilisé pour mesurer le taux d'humidité résiduelle des feuilles.

De préférence, dans le protocole susmentionné, on fait macérer la poudre dans une solution d'alcool de canne à sucre à une température de 25°C à 30°C, de préférence 30°C, pendant environ 21 jours, avec mise sous agitation lente tous les jours pendant 12h.

<u>Préparation de la teinture mère d'un extrait de feuilles d'une plante de *Neurolaena lobata* constituant l'échantillon H2</u>

La teinture mère H2 d'un extrait de feuilles d'une plante de Neurolaena *lobata* est obtenue en réalisant les étapes de procédé suivantes :

- On récolte les feuilles de *Neurolaena lobata,*
- On sèche lesdites feuilles,

- On fait macérer 100 g de feuilles séchées dans 1L d'une solution d'éthanol pur pendant 7 jours jusqu'à obtention d'un macérat,
- On filtre ledit macérat sur célite jusqu'à obtention d'un filtrat,
- On concentre par évaporateur rotatif ledit filtrat.

On sèche sous pression ledit filtrat concentré, notamment à l'aide d'une rampe à vide, jusqu'à obtention de l'échantillon H2.

Protocole d'analyse de l'inhibition de la DHODH par la composition de l'invention.

Afin de vérifier l'inhibition de la DHODH par la composition thérapeutique de l'invention, deux solutions mères, nommées respectivement H1 et H2, ont été préparées.

La solution mère H1 est obtenue en mettant en œuvre le procédé susmentionné après récolte des feuilles d'une plante de *Neurolaena lobata.*

La solution mère H2 est obtenue en mettant en œuvre le procédé susmentionné.

Afin de tester l'impact de l'échantillon sur l'inhibition de la DHODH et de connaître « l'effet dose réponse », chaque échantillon H1 et H2 a été dilué dans un tampon de DMSO.

Les dilutions des échantillons H1 et H2 ont permis d'obtenir les concentrations suivantes : en µg d'échantillon / mL total de solution dans le puit : 0,01 µg/mL ; 0,1 µg/mL ; 1µg/mL ; 10 µg/mL ; 100 µg/mL ; 1000 µg/mL indiquées dans respectivement dans le tableau 1 et le tableau 2 ci-dessous.

Pour démarrer le protocole, chaque dilution de l'échantillon H1 ou H2 a été mise en présence de l'enzyme rh DHODH et du mélange de substrat au sein d'un puit. Afin d'obtenir une moyenne de DO mesurée pour une concentration d'échantillon H1 ou H2, on a réalisé des tripliqua pour chacune des concentrations de dilution de H1 et H2.

Plus spécifiquement, pour obtenir les résultats ci-dessous, on a réalisé les étapes suivantes du protocole :

- On ajoute, dans chaque puit comportant les dilutions des échantillons H1 ou H2, l'enzyme rh DHODH préparée le jour même dans une solution tampon test,
- On laisse en présence l'enzyme et l'échantillon pendant 6 min à 37°C,
- On ajoute ensuite 50 µL du mélange de substrat coloré susmentionné, ceci consiste en le temps 0,
- On mesure dans chaque puits par lecture immédiate la DO à 600 nm, à intervalle régulier, toutes les 55 secondes, pendant 275 secondes.

La quantité d'enzyme ajoutée dans chaque puit est la même. Dans le protocole, on ajoute l'enzyme dans le puit de sorte à avoir une concentration de 0,06 µg d'enzyme rh DHODH /mL de solution totale dans le puit.

Le tableau 1 ci-dessous montre les résultats obtenus pour l'échantillon H1 :

*Tableau 1 : Inhibition de l'activité de la DHODH par l'échantillon H1*

| Temps (s) | Concentration des échantillons H1 en µg de H1/ mL de solution totale dans le puit | | | | | | Contrôle négatif |
|---|---|---|---|---|---|---|---|
| | 0,01 µg/ml | 0,1 µg/ml | 1 µg/ml | 10 µg/ml | 100 µg /ml | 1000 µg/ml | E+S+T |
| 0 | 0,324 | 0,335 | 0,347 | 0,356 | 0,364 | 0,371 | 0,349 |
| 55 | 0,315 | 0,326 | 0,335 | 0,338 | 0,346 | 0,357 | 0,321 |
| 110 | 0,307 | 0,345 | 0,324 | 0,335 | 0,345 | 0,352 | 0,241 |
| 165 | 0,246 | 0,258 | 0,264 | 0,272 | 0,280 | 0,303 | 0,211 |
| 220 | 0,233 | 0,243 | 0,257 | 0,255 | 0,273 | 0,283 | 0,192 |
| 275 | 0,232 | 0,241 | 0,254 | 0,255 | 0,254 | 0,277 | 0,141 |
| Δ | 0,092 | 0,094 | 0,093 | 0,101 | 0,100 | 0,094 | 0,208 |
| % activité | 44,231 | 45,192 | 44,711 | 48,558 | 48,077 | 45,192 | 100,000 |
| % inhibition | 55,769 | 54,808 | 55,289 | 51,442 | 51.923 | 54,808 | 0,000 |

Dans le tableau 1, la première colonne donne les intervalles de temps de prise de mesure de la DO à 600 nm. En d'autres termes, ceci correspond au temps de mise en contact de l'échantillon H1 avec la rh DHODH en présence du mélange de substrat.

Dans le tableau 1, la deuxième ligne indique la concentration de l'échantillon H1 dans le puit. Cette concentration est exprimée en μg d'échantillon H1/mL de solution totale dans le puit.

Chaque colonne du tableau 1 indique la valeur moyenne de DO mesurée à 600 nm des tripliqua pour une même concentration d'échantillon H1, et pour un temps défini de mise en contact avec la rh DHODH et son substrat.

Par exemple, tel que visible dans le tableau 1, après 110 secondes de mise en contact de l'échantillon H1 à une concentration de 0,1 μg/mL avec la rh DHODH, et le mélange de substrat coloré, la valeur moyenne de la DO mesurée sur les trois puits, de contenance identique, est de 0,315.

La ligne du signe Δ du tableau 1 indique la différence entre la valeur moyenne de la DO mesurée à 0 seconde de mise en présence et la valeur moyenne de DO mesurée à 275 secondes de mise en présence entre l'échantillon H1, la rh DHODH et son substrat.

Le signe Δ donne la diminution de la DO entre 0 et 275 secondes de mise en présence, c'est-à-dire la capacité de transformation de la DHO en ORO par l'activité effective de la DHODH.

Dans le tableau 1, les deux dernières lignes donnent, pour chaque concentration d'échantillon de H1, le pourcentage d'activité de transformation de la DHO coloré en ORO par l'activité de la rh DHODH, ainsi que le pourcentage d'inhibition de l'activité de la rhDHODH par l'échantillon H1.

Le % d'activité est calculé de la manière suivante, pour une concentration donnée d'échantillon H1 (par la suite H2) :

$$\% \text{ d'activité} = (\Delta H1 X 100) / \Delta \text{ du contrôle négatif.}$$

Par exemple, pour l'échantillon H1 à 0,01 μg/mL : le %d'activité = (0,092 X 100) / 0,208 = 44,231.

Le % d'inhibition est calculé par la formule suivante : 100- la valeur du % d'activité.

Pour valider l'activité effective de la rh DHODH de transformation de son substrat DHO lors de l'expérience, un contrôle négatif a été réalisé en duplicata. Ce contrôle négatif est essentiel pour valider l'activité de la DHODH sur son substrat et pour déterminer le % d'activité et le % d'inhibition des échantillons.

La colonne de contrôle négatif montre les valeurs moyennes de DO mesuré, à 600 nm, aux différents intervalles de mesures en seconde.

Le contrôle négatif contient : rh DHODH noté « E » dans le tableau 1 à une concentration de 0,06 μg/ mL de solution totale dans le puit, avec 50 μL du mélange de substrat coloré noté « S » et, en remplacement de l'échantillon H1, uniquement une solution de tampon de DMSO notée « T ».

Les résultats montrent qu'entre 0 et 275 secondes, la valeur moyenne de la DO mesurée à 600 nm diminue.

En conséquence, le substrat coloré DHO est bien transformé en ORO, par l'activité de réduction par l'enzyme rh DHODH. L'enzyme rh DHODH est bien fonctionnelle vis-à-vis du mélange substrat. De plus, ni la solution tampon de DMSO, ni la solution de tampon test dans laquelle est dilué le substrat DHO n'impacte l'activité de déshydrogénation par l'enzyme rh DHODH.

Tel que visible dans le tableau 1, l'échantillon H1 de l'invention inhibe l'activité de la DHODH pour son substrat. Pour toutes les concentrations de H1 testées, le pourcentage d'inhibition est compris entre 51% et 56%. Il est également constaté que pour les concentrations testées, une augmentation de la concentration de l'échantillon H1 n'est pas synonyme d'une augmentation de la valeur du pourcentage d'inhibition à l'encontre de l'activité de la DHODH.

Le même protocole et les mêmes mesures de DO ont permis de quantifier le pourcentage d'inhibition de l'échantillon H2.

Le tableau 2 ci-dessous montrent les résultats obtenus pour les différentes concentrations testées de l'échantillon H2

*Tableau 2 : Inhibition de l'activité de la DHODH par l'échantillon H2*

| Temps (s) | Concentration des échantillons H1 en μg de H1/ mL de solution totale dans le puit | | | | | | Contrôle négatif |
|---|---|---|---|---|---|---|---|
| | 0,01 μg/ml | 0,1 μg/ml | 1μg/ml | 10 μg/ml | 100 μg /ml | 1000 μg/ml | E +S+T |
| 0 | 0,312 | 0,330 | 0,337 | 0,347 | 0,358 | 0,366 | 0,349 |
| 55 | 0,299 | 0,311 | 0,320 | 0,333 | 0,340 | 0,350 | 0,321 |
| 110 | 0,283 | 0,255 | 0,297 | 0,311 | 0,320 | 0,325 | 0,241 |
| 165 | 0,254 | 0,269 | 0,279 | 0,282 | 0,297 | 0,303 | 0,211 |
| 220 | 0,235 | 0,244 | 0,258 | 0,260 | 0,270 | 0,304 | 0,192 |

(suite)

| Temps (s) | Concentration des échantillons H1 en µg de H1/ mL de solution totale dans le puit | | | | | | Contrôle négatif |
|---|---|---|---|---|---|---|---|
| | 0,01 µg/ml | 0,1 µg/ml | 1µg/ml | 10 µg/ml | 100 µg /ml | 1000 µg/ml | E +S+T |
| 275 | 0,213 | 0,224 | 0,230 | 0,240 | 0,251 | 0,296 | 0,141 |
| Δ | 0,099 | 0,106 | 0,107 | 0,107 | 0,107 | 0,07 | 0,208 |
| % activité | 47,596 | 50,961 | 51,440 | 51,440 | 51,440 | 33,654 | 100,000 |
| % inhibition | **52,404** | 49,039 | 48,56 | 48,56 | 48,56 | **66,346** | 0,000 |

Tel que visible dans le tableau 2, l'échantillon H2 de l'invention inhibe l'activité de la DHODH.

Pour toutes les concentrations de H2 testées, le pourcentage d'inhibition est compris entre 48% et 67%.

A la différence de l'échantillon H1, il semble y avoir un effet dose-réponse. En effet, pour une concentration de 1000 µg/mL, le pourcentage d'inhibition semble significativement plus élevé que pour une concentration de 0,01 µg/mL.

En d'autres termes, quand on augmente la concentration de l'échantillon H2 en présence de l'enzyme, le pourcentage d'inhibition augmente. L'échantillon H2 diffère de l'échantillon H1 par le protocole de préparation de l'extrait des feuilles de *Neurolaena lobata.* Il semblerait par conséquent que les éléments responsables de l'activité inhibitrice de la rh DHODH soit concentrés dans les feuilles et que, selon le mode de préparation de la teinture mère de feuilles, l'activité inhibitrice soit différente.

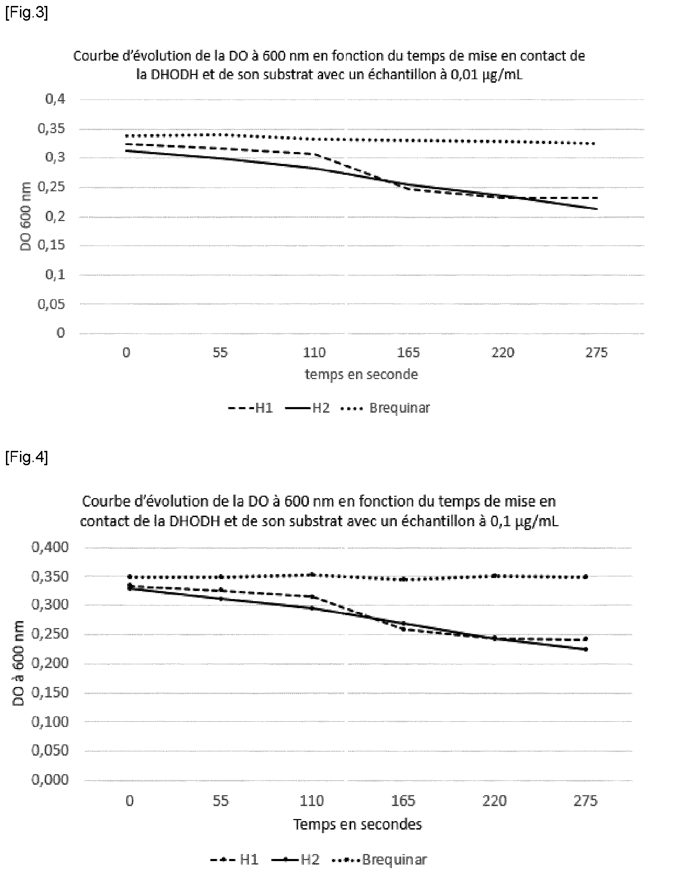

Les figures 3 à 5 illustrent l'évolution de la densité optique mesurée à 600 nm au cours du temps, pour des échantillons de H1, H2 ou de brequinar à une concentration donnée.

La courbe de la figure 3 présente la mesure des valeurs de DO à 600 nm, au cours du temps, pour des échantillons à une concentration de 0,01 µg/ml totale dans le puit.

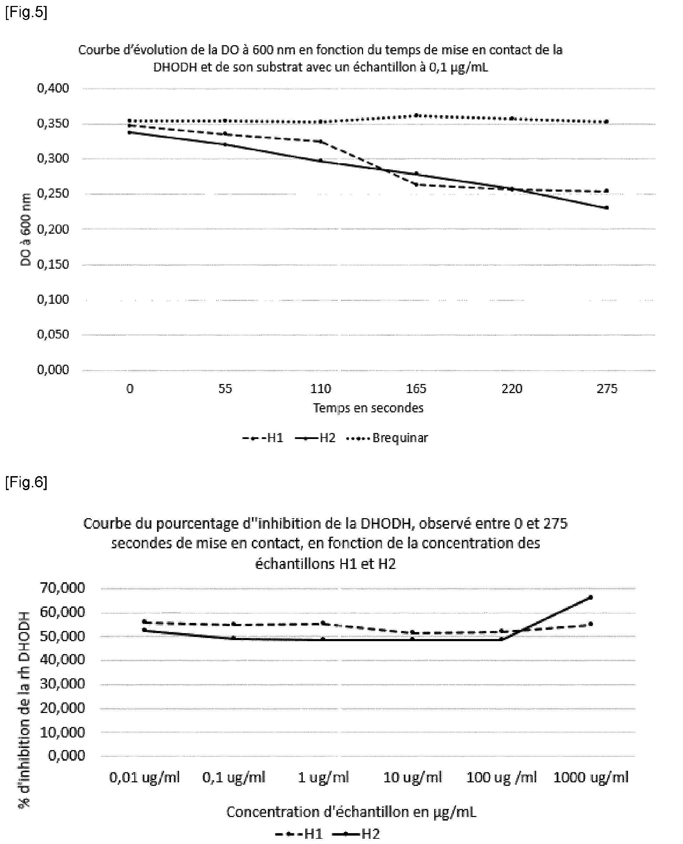

De la même manière, la courbe de la figure 4 est pour des échantillons à une concentration de 0,1 µg/mL et la courbe de la figure 5 est pour des échantillons à une concentration de 1 µg/mL.

Il est à noter que, dans tous les cas, peu importe la concentration de l'échantillon, pour le brequinar, inhibiteur connu de la DHODH, la DO à 600 nm reste quasiment stable au cours du temps. Le brequinar est donc bien un inhibiteur de la DHODH.

Pour les échantillons H1 et H2, il est observé une diminution de la DO mesurée à 600 nm. Cette diminution est synonyme de l'activité de la rh DHODH. Néanmoins, il est constaté que pour chacun des échantillons, la diminution de la DO est franche à partir de 110 secondes de mise en contact.

Entre 0 et 110 secondes, la DO mesurée à 600 nm est plutôt stable. Ce constat semble signifier que l'enzyme DHODH n'est pas active immédiatement. Dans les 110 premières secondes, la DHODH ne réalise pas ou peu la réaction de déshydrogénation, lorsqu'elle est en présence des échantillons H1 ou H2. Les échantillons H1 et H2 semblent donc ralentir le démarrage de l'activité de la DHODH vis-à-vis de son substrat.

Afin de valider, effectivement, l'activité de l'enzyme rh DHODH de réduction de son substrat, on a également réalisé un contrôle positif en duplicata. Les puits de contrôle positif comportent, en remplacement de l'échantillon H1 ou H2 dilué, du brequinar, qui est un inhibiteur connu de l'activité de la DHODH.

En parallèle à la mesure de DO des échantillons, on a mesuré la DO d'une solution de brequinar diluée dans un tampon DMSO en présence de la rh DHODH, et de son substrat.

De la même manière que pour les échantillons, on a mesuré l'action de différentes concentrations de la solution brequinar sur l'inhibition de la rh DHODH.

Afin de valider l'activité de la rh DHODH sur son substrat, il est réalisé en triplicata un contrôle positif, en parallèle du protocole de mesure de l'inhibition de la DHODH par le brequinar. Les puits de contrôle positif comportent la rh DHODH dit « E », et son mélange de substrat dit « T » ainsi, qu'en remplacement du brequinar, du tampon DMSO dit « T ».

Le tableau 3 ci-dessous montrent les résultats obtenus pour les différentes concentrations testées de l'échantillon de brequinar

*Tableau 3 : Inhibition de l'activité de la DHODH par le brequinar*

| Temps (s) | Concentration des échantillons de brequinar en µg de brequinar/mL de solution totale dans le puit | | | | Contrôle positif |
|---|---|---|---|---|---|
| | 0,001 µg/ml | 0,01µg/ml | 0,1µg/ml | 1 µg/ml | E +S+T |
| 0 | 0,335 | 0,344 | 0,349, | 0,354 | 0,345 |

(suite)

| Temps (s) | Concentration des échantillons de brequinar en μg de brequinar/mL de solution totale dans le puit | | | | Contrôle positif |
|---|---|---|---|---|---|
| | 0,001 μg/ml | 0,01μg/ml | 0,1μg/ml | 1 μg/ml | E +S+T |
| 55 | 0,340 | 0,345 | 0,348 | 0,354 | 0,321 |
| 110 | 0,333 | 0,339 | 0,353 | 0,352 | 0,241 |
| 165 | 0,331 | 0,336 | 0,345 | 0,361 | 0,211 |
| 220 | 0,328 | 0,348 | 0,350 | 0,357 | 0,192 |
| 275 | 0,326 | 0,329 | 0,349 | 0,353 | 0,141 |
| Δ | 0,013 | 0,015 | 0,000 | 0,001 | 0,208 |
| % activité | 6,250 | 7,211 | 0,000 | 0,000 | 100,000 |
| % inhibition | 93,750 | 92,789 | 100,000 | 99,999 | 0,000 |

Dans le tableau 3, l'activité de la rh DHODH de transformation de son substrat est validée par le contrôle positif. En effet, au cours du temps, on observe effectivement une diminution de la DO mesurée à 600 nm. Cette diminution résulte bien de la transformation de la DHO en ORO, par l'activité de déshydrogénation de la rh DHODH. Ni le tampon DMSO, ni le tampon test, n'impactent l'activité de l'enzyme rh DHODH. L'enzyme rh DHODH est donc fonctionnelle dans ce protocole de mesure de l'inhibition de la DHODH par le brequinar.

Le tableau 3 montre que les différentes concentrations de brequinar testées présentent une activité d'inhibition de la rh DHODH comprise entre 92% et 100%.

Pour mettre en avant l'activité d'inhibition des échantillons H1 et H2, la figure 6 montre la courbe du pourcentage d'inhibition des échantillons H1 et H2 l'un par rapport à l'autre, en fonction de leur concentration, après 275 secondes de mise en contact avec la DHODH et son substrat.

Sur la figure 6, il est constaté que, pour des concentrations allant de 0,01 à 1000 μg/mL, H1 présente un pourcentage d'inhibition de la rh DHODH relativement stable.

Au contraire, H2 présente une augmentation significative de son pourcentage d'inhibition pour une concentration dépassant les 100μg/mL. En particulier, pour une concentration de 1000μg de H2/ mL de solution totale dans le puit, le pourcentage d'inhibition de la rh DHODH atteint avantageusement 66,3%.

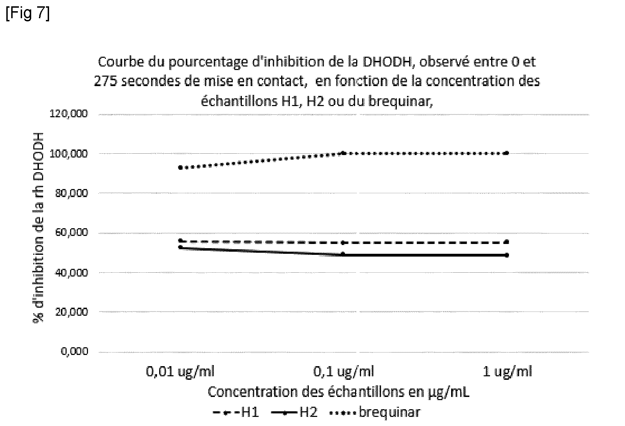

De la même manière, la figure 7 montre l'évolution du pourcentage d'inhibition des échantillons H1, H2 et du brequinar en fonction de leur concentration après 275 secondes de mise en contact avec la DHODH et son substrat. Il est constaté que les échantillons H1 et H2, tout comme le brequinar, sont des inhibiteurs de l'activité de la DHODH sur substrat. En effet, bien que l'inhibition de la DHODH par les échantillons H1 et H2 ne soit pas à 100% et aussi performante que le brequinar, elle existe.

En conséquence, au regard des résultats obtenus, les échantillons de teintures mères de *Neurolaena lobata* H1 et H2 d'origine naturelle présentent effectivement, et de manière significative, une activité inhibitrice de l'enzyme DHODH.

Ainsi, la composition thérapeutique de l'invention comportant une teinture mère d'un extrait de feuilles d'une plante appartenant au genre « *Neurolaena »* et à l'espèce « *lobata* » a pour effet d'inhiber l'activité de la DHODH.

De ce fait, une composition thérapeutique comprenant l'un ou l'autre de ces échantillons, c'est-à-dire une teinture mère de *Neurolaena lobata* à base de feuille est un produit naturel, non toxique prometteur pour traiter des maladies dont la cible thérapeutique est l'inactivation de la DHODH.

En particulier, la composition thérapeutique de l'invention est une piste envisageable pour traiter des maladies résultant de l'infection par un pathogène virale, notamment les virus à génome à ARN.

Des tests in vitro ont également été menés pour démontrer l'efficacité antivirale et virucide, ainsi que pour déterminer l'effet inhibiteur sur la libération de certaines cytokines, de la composition thérapeutiques à base de teinture mère de *Neurolaena lobata.*

Préparation des échantillons à tester :

Plusieurs échantillons ont été préparés à partir de feuilles de la plante *Neurolaena lobata* pour la conduite de ces tests.

Les échantillons sont notés « TOTUM ».

Le « TOTUM 3 » correspond à une teinture mère obtenue à partir de feuilles de *Neurolaena lobata* ; il a été préparé de la manière suivante :

i) Une masse de 3.6 kg de feuilles sèches de *Neurolaena lobata* a été mélangée avec un volume de 225L d'alcool de canne à sucre à 50°, ce qui correspond à une concentration massique de 16g de feuilles sèches de *Neurolaena lobata* par litre d'alcool de canne à sucre ; de manière plus générale, on prépare un mélange présentant une concentration massique comprise entre 15 et 20 g/L, de préférence entre 16 et 17 g/L et, plus préférentiellement, égale à 16g de feuilles sèches de *Neurolaena lobata* par litre d'alcool de canne à sucre à 50° ;

Préférentiellement, tout comme pour la préparation de l'échantillon H1 évoquée ci-dessus, on peut sécher les feuilles sous un courant d'air chaud, à une température de préférence inférieure à 40°C, pendant environ 120 heures, jusqu'à ce qu'elles présentent un taux d'humidité résiduelle de l'ordre de 6 à 7%. Le taux d'humidité peut être déterminé de la même manière que pour H1 également.

ii) Le mélange est laissé à macérer sous agitation pendant environ 21 jours, à une température de l'ordre de 30°C, avec mise sous agitation lente tous les jours pendant 12h ;

iii) Après macération, le mélange est filtré sur « cartouches » ou poches de filtration de porosité 50-75 $\mu$m et on obtient un filtrat et un rétentat ;

iv) prélèvement d'un volume de 3L de filtrat

v) évaporation de l'alcool contenu dans ce dernier par évaporateur rotatif, en trois fois, jusqu'à obtention d'une solution aqueuse présentant un volume environ égal à 1L.

vi) Congélation de la solution aqueuse ainsi obtenue, puis lyophilisation.

On obtient finalement un lyophilisat présentant une masse égale à 12,3g.

Le « TOTUM 4 » est un échantillon qui est dilué à partir de la teinture mère.

Plus particulièrement, pour l'obtention de cet échantillon, à l'étape iv) du protocole d'obtention du TOTUM 3 ci-dessous, au lieu de prélever 3L de filtrat, on prélève 0.75L de filtrat que l'on dilue dans un volume égal à 2,25L d'eau. On obtient alors une solution hydroalcoolique présentant un volume total égal à 3L. De manière générale, on effectue une dilution au ¼ du filtrat liquide, ou teinture mère, obtenu(e) à l'étape iii) ci-dessus.

Ensuite, les étapes suivantes pour l'obtention du TOTUM 4 sont similaires à celles qui sont mises en œuvre pour l'obtention du TOTUM 3 et qui ont été décrites ci-dessus, à savoir :

v) évaporation de l'alcool contenu dans la solution hydroalcoolique, présentant préférentiellement un volume égal à 3L, par évaporateur rotatif, par exemple en trois fois, jusqu'à obtention d'une solution aqueuse, celle-ci pouvant présenter un volume environ égal à 1L ;

vi) Congélation de la solution aqueuse ainsi obtenue, puis lyophilisation.

On obtient finalement un lyophilisat présentant une masse égale à 5,6 g et correspondant à un extrait sec d'une teinture mère diluée (au quart) de feuilles sèches de *Neurolaena lobata.*

A titre de témoin négatif, un échantillon dénommé « TOTUM 2 » a été préparé, à partir de pulpe de bananes sèches *Musa sapentium.*

Plus particulièrement, pour l'obtention de cet échantillon, 150g de pulpe de bananes sèches ont été diluées dans un volume de 5L d'alcool de canne à sucre à 50°.

Le mélange est mis à macérer pendant une durée d'environ 5 jours, avec une mise sous agitation pendant 2 à 3h par jour, avant d'être filtré sur papier de porosité comprise entre 10 et 20$\mu$m, pour obtenir 4L de filtrat hydroalcoolique.

On effectue ensuite une évaporation du filtrat par évaporateur rotatif, jusqu'à l'obtention d'un extrait sec, d'une masse égale à 6g.

Evaluation de l'inhibition de l'expansion du virus SARS-CoV2 responsable de la Covid 19 lors du traitement de cellules épithéliales pulmonaires humaines (Calu-3) et de cellules rénales (VeroE6-TMPRSS2) par les TOTUM 2, 3 et 4

La souche de virus SARS-CoV2 qui a été utilisée lors de la conduite de ces tests est la souche Européenne (une mutation de la souche originelle de Wuhan en D614G), qui correspond à la souche SARS-CoV-2 de référence Slovakia/SK-BMC5/2020.

La souche virale a été fournie par la plateforme European Virus Archive goes Global (Evag) (https://www.european-virus-archive.com/).

La souche virale de SARS-Cov2 a été amplifiée et titrée sur la lignée cellulaire VeroE6 TMPRSS2 par Oncodesign.

Deux lignées cellulaires ont été utilisées dans ces tests d'évaluation : il s'agit des lignées suivantes :

- Calu-3, adénocarcinome pulmonaire humain (origine ATCC - American Type Culture Collection) ;
- Vero E6-TMPRSS2, cellules épithéliales de rein de primate non humain (origine NIBSC - National Institute for Biological Standards and Controls, UK).

Le modèle cellulaire Calu-3 est déjà bien décrit dans la littérature pour le Sars-CoV (voir C.-T. K. Tseng, J. Tseng, L. Perrone, M. Worthy, V. Popov, and C. J. Peters, "Apical entry and release of severe acute respiratory syndrome-associated coronavirus in polarized Calu-3 lung epithelial cells," J Virol, vol. 79, no. 15, pp. 9470-9479, Aug. 2005, doi: 10.1128/JVI.79.15.9470-9479.2005).

Les cellules Calu-3 ont été cultivées en monocouche à 37°C dans une atmosphère humidifiée (5% CO2, 95% air) dans le milieu de culture cellulaire correspondant (MEM + 1% Pyruvate + 1% glutamine + 10% Sérum Fœtal Bovin).

Les cellules Vero E6-TMPRSS2 ont été cultivées en monocouche à 37°C dans une atmosphère humidifiée (5 % de $CO_2$, 95 % d'air) dans le milieu de culture cellulaire correspondant (DMEM + 1 % de pyruvate + 1 % de cocktail d'antibiotiques (pénicilline, streptomycine et généticine) + 2 % de sérum bovin fœtal).

Les cellules de ces deux lignées cellulaires sont adhérentes aux flacons en plastique. Pour les procédures de passage des cellules, ces dernières ont été détachées du flacon de culture par un traitement de 20 minutes (pour les cellules de la lignée Calu) et de 5 minutes (pour les cellules de la lignée Vero) avec de la trypsineversène et neutralisées par l'ajout de milieu de culture complet. Pour l'étude, les cellules ont été déposées sur des plaques de 96 puits.

Les cellules ont été comptées et leur viabilité a été évaluée en utilisant le compteur de cellules Vi-cell.

Dans une première série de tests, notée « CAS1 », les cellules des deux lignées cellulaires susmentionnées sont mises en contact avec les composés à tester (TOTUM 2, 3 et 4 notamment), pendant une durée de 24h, avant exposition à la souche virale de SARS-CoV-2. Cette première série « CAS 1 » permet d'étudier l'effet antiviral, autrement dit les cellules sont traitées par le composé avant d'être infectées.

Dans une deuxième série de tests, notée « CAS 2 », la souche virale de SARS-CoV-2 est mise en contact avec les différents composés à tester, dont les TOTUMs 2, 3 et 4, pendant une durée de 30 min à température ambiante, avant mise en contact des cellules avec le virus. Cette deuxième série « CAS 2 » permet d'étudier l'effet virucide, autrement dit le virus est mis en contact avec le composé avant une mise en contact avec les cellules.

Protocole de tests pour les tests CAS 1 avec les lignées cellulaires Calu-3 et Vero E6 TMPRSS2

Les cellules ont été comptées et leur viabilité évaluée à l'aide de l'analyseur cellulaire Vi-CELL.

Les cellules ont été ensemencées pour atteindre la confluence :

- Vero E6 TMPRSS2 - 30,000 cellules/puits ;
- Calu-3 - 90,000 cellules/puits.

A partir des lyophilisats et extraits secs des TOTUMs 2, 3 et 4, des solutions mères sont préparées dans du DMSO à 10mg/mL. A partir de ces solutions mères, sept concentrations de composés à tester ont été préparées dans un milieu de croissance complet et ajoutées aux cellules : 10000, 3333, 1111, 370, 123, 41, 14 ng/mL.

Le premier réplicat biologique (N=1) a été effectué avec ces concentrations.

Le deuxième réplicat biologique (N=2) a été effectué avec des concentrations différentes. En effet, les concentrations testées ont été ajustées après l'analyse des résultats du premier réplicat biologique.

Ainsi pour le réplicat N=2, les concentrations suivantes ont été utilisées : 100000, 33333, 11111, 3704, 1235, 412 et 137 ng/mL pour le TOTUM 3 et 20000, 6667, 2222, 741, 247, 82 et 27 ng/mL pour les TOTUMs 2 et 4.

En tant que composé contrôle de référence, ou témoin positif, le métabolite actif du remdesivir a été utilisé. Sept concentrations de remdesivir (20000, 6667, 2222, 741, 247, 82, 27 nM), ont été préparées et ajoutées aux cellules.

Le métabolite actif du remdesivir a été fourni par Oncodesign, sous la forme d'une solution mère à 20mM dans du DMSO.

Les plaques ont été incubées pendant 24 h à 37°C.

Ensuite, un volume de 10 µL de préparation virale équivalente à une MOI (Multiplicity Of Infection) = 0,01 a été ajouté et incubé à 37°C pendant 48 h pour les cellules VeroE6 TMPRSS2 et 72 h pour les cellules Calu-3.

Une fraction (50 µL) des surnageants a été recueillie puis stockée à une température égale à -20°C pour déterminer la charge virale.

Une fraction (~200 µL en trois aliquotes : 2x50 µL + le volume restant) des surnageants a été recueillie puis stockée à une température égale à -20°C pour le dosage des cytokines.

Protocole de tests pour les tests CAS 2 avec les lignées cellulaires Calu-3 et Vero E6 TMPRSS2

Les cellules ont été comptées et leur viabilité a été évaluée à l'aide de l'analyseur cellulaire Vi-CELL.

Les cellules ont été ensemencées pour atteindre la confluence :

- Vero E6 TMPRSS2 - 35 000 cellules/puits ;
- Calu-3 - 80 000 cellules/puits.

A partir des lyophilisats et extraits secs des TOTUMs 2, 3 et 4, sept concentrations de composés à tester ont été préparées dans un milieu de croissance frais : 10000, 3333, 1111, 370, 123, 41, 14 ng/mL.

Le premier réplicat biologique (N=1) a été effectué avec ces concentrations.

Tout comme pour le CAS 1, le deuxième réplicat biologique (N=2) a été effectué avec des concentrations différentes.

Ainsi pour le réplicat N=2, les concentrations suivantes ont été utilisées : 100000, 33333, 11111, 3704, 1235, 412 et 137 ng/mL pour le TOTUM 3 et 20000, 6667, 2222, 741, 247, 82 et 27 ng/mL pour les TOTUMs 2 et 4.

De même, sept concentrations du contrôle de référence, ou témoin positif, le métabolite actif du remdesivir (20000, 6667, 2222, 741, 247, 82, 27 nM), ont été préparées.

Un volume de 10 μL de préparation virale équivalente à une MOI = 0,01 a été mélangé avec les composés à tester et incubé à température ambiante pendant 30 min.

Le mélange composé/virus a ensuite été ajouté aux cellules.

Les cellules ont été incubées à 37°C pendant 48 h pour les cellules VeroE6-TMPRSS2 et 72 h pour les cellules Calu-3.

Une fraction (50 μL) des surnageants a été recueillie puis stockée à -20°C pour déterminer la charge virale.

Une fraction (~200 μL en trois aliquotes : 2x50 μL + le volume restant) des surnageants a été recueillie puis stockée à -20°C pour dosage des cytokines.

A noter : une plaque sans virus a été réalisée pour évaluer la cytotoxicité des composés testés sur les deux types cellulaires. La viabilité cellulaire a été évaluée par le test CellTiter Glo pour toutes les conditions selon les recommandations du fabricant (Promega, G7570).

Pour les CAS 1 et CAS 2, la quantification de la charge virale par RTqPCR, ciblant le gène ORF1ab viral, a été réalisée à la fin de l'expérience.

L'extraction de l'ARN viral a été réalisé par la trousse Macherey Nagel Viral RNA et l'ARN a été congelé à -80°C jusqu'à réalisation de la RT-qPCR.

La RT-qPCR complète a été réalisée à l'aide de la trousse SuperScript™ One-Step qRT-PCR System, avec des amorces et des conditions de qRT-PCR ciblant le gène ORF1ab. Les amplifications ont été réalisées avec un appareil Bio-Rad CFX384™ et du logiciel correspondant.

Toujours pour les CAS 1 et CAS 2, le test de cytotoxicité ou test de viabilité cellulaire luminescent CellTiter-Glo® a été réalisé à la fois sur une plaque témoin (sans virus) et sur les plaques traitées et infectées pour évaluer la cytotoxicité des échantillons testés.

Le test de viabilité cellulaire luminescent CellTiter-Glo® est une méthode homogène de détermination du nombre de cellules viables en culture basée sur la quantification de l'ATP présent, un indicateur de cellules métaboliquement actives.

La méthode a été utilisée pour les cellules VeroE6-TMPRSS2 et Calu-3 en l'absence de virus pour établir la cytotoxicité de chacun des composés qui ont été testés.

La méthode a également été utilisée pour les cellules VeroE6-TMPRSS2 en présence de virus 48h après l'infection en cas d'effets cytopathogènes (présence de virus actif) ; la présence de virus dans le modèle cellulaire Vero se traduit par des effets cytopathogènes après utilisation de la machinerie cellulaire alors que le virus est produit en continu dans le modèle Calu.

Le test a été réalisé selon le protocole du fournisseur.

Après avoir retiré tout le surnageant pour les réactions PCR et les dosages des cytokines, ajouter 100μL de milieu cellulaire frais à 100μL de réactif et incuber jusqu'à l'enregistrement de la luminescence, au moins 15 min après le mélange.

Pour les CAS 1 et 2, le dosage des cytokines, plus particulièrement de l'IL 6, du MCP1 et de l'IP10 ont été effectués par ELISA à l'aide de trousses commerciales sur des surnageants de culture cellulaire recueillis 48h et 72h post-infection, respectivement pour les lignées cellulaires Vero et Calu-3.

Résultats : évaluation de la cytotoxicité des TOTUMs (sans virus)

La toxicité des échantillons à tester « TOTUMs » et du métabolite actif du remdesivir sur des cellules Calu-3 non exposées au virus a été évaluée par une mesure de viabilité cellulaire après une exposition de 96h aux composés.

Les résultats sont exposés dans les tableaux ci-après, la viabilité cellulaire étant exprimée en % par rapport aux cellules non traitées, après exposition aux composés de la lignée cellulaire Calu-3, ET correspondant à l'écart-type :

| | Métabolite actif du remdesivir | |
|---|---|---|
| **(nM)** | **Moy** | **ET** |
| **27** | 91 | 17 |

(suite)

| (nM) | Métabolite actif du remdesivir | |
|---|---|---|
| | Moy | ET |
| 82 | 94 | 14 |
| 247 | 116 | 22 |
| 741 | 103 | 34 |
| 2222 | 111 | 20 |
| 6667 | 96 | 11 |
| 20000 | 116 | 18 |

| cc (ng/mL) | TOTUM 2 | | TOTUM 3 | | TOTUM 4 | |
|---|---|---|---|---|---|---|
| | Moy | ET | Moy | ET | Moy | ET |
| 14 | 131 | 11 | 120 | 8 | 102 | 31 |
| 41 | 93 | 20 | 64 | 18 | 109 | 23 |
| 123 | 96 | 4 | 111 | 17 | 106 | 25 |
| 370 | 99 | 51 | 110 | 4 | 138 | 2 |
| 1111 | 103 | 39 | 90 | 4 | 103 | 31 |
| 3333 | 124 | 25 | 99 | 11 | 120 | 29 |
| 10000 | 113 | 20 | 95 | 7 | 110 | 22 |

Après traitement avec le métabolite actif du remdesivir, les résultats sont similaires entre les réplicats biologiques avec une viabilité cellulaire moyenne allant de 91% (27 nM, N=1) à 116% (247nM et 2 0000 nM, N=1).

Lors du réplicat biologique N=1, la viabilité cellulaire après traitement avec les TOTUMS 2, 3 et 4, pour les sept concentrations allant de 14 ng/mL à 10 000 ng/mL, était similaire à celle obtenue après traitement au métabolite actif du remdesivir.

Des résultats similaires ont également été obtenus pour les TOTUMs 2 et 4 lors du réplicat biologique N=2 (voir tableaux ci-dessous), pour les concentrations testées allant de 137 ng/mL à 100 000 ng/mL.

| (nM) | Métabolite actif du remdesivir | |
|---|---|---|
| | Moy | ET |
| 27 | 112 | 3 |
| 82 | 108 | 2 |
| 247 | 109 | 5 |
| 741 | 106 | 2 |
| 2222 | 113 | 2 |
| 6667 | 113 | 1 |
| 20000 | 104 | 2 |

| cc (ng/mL) | TOTUM 3 | |
|---|---|---|
| | Moy | ET |
| 137 | 102 | 3 |
| 412 | 93 | 3 |
| 1 235 | 98 | 3 |
| 3 704 | 99 | 5 |
| 11 111 | 96 | 4 |
| 33 333 | 84 | 3 |
| 100 000 | 1 | 0 |

| cc (ng/mL) | TOTUM 2 Moy | TOTUM 2 ET | TOTUM 4 Moy | TOTUM 4 ET |
|---|---|---|---|---|
| 27 | 106 | 4 | 105 | 1 |
| 82 | 101 | 4 | 96 | 2 |
| 247 | 104 | 3 | 101 | 4 |
| 741 | 104 | 1 | 103 | 4 |
| 2 222 | 103 | 4 | 100 | 3 |
| 6 667 | 103 | 4 | 102 | 2 |
| 20 000 | 97 | 2 | 99 | 0 |

Toutefois, il est à noter qu'une diminution de la viabilité cellulaire a été observée pour le TOTUM 3, pour une concentration de 100 000 ng/mL.

La toxicité des TOTUMs 2, 3 et 4 et du métabolite actif du remdesivir sur des cellules VeroE6-TMPRSS2 non exposées au virus a également été évaluée par une mesure de viabilité cellulaire après une exposition de 72h aux composés.

Les résultats sont exposés dans les tableaux ci-après, la viabilité cellulaire étant exprimée en % par rapport aux cellules non traitées, après exposition aux composés de la lignée cellulaire VeroE6, ET correspondant à l'écart-type :

| | Métabolite actif du remdesivir | | | Métabolite actif du remdesivir | |
|---|---|---|---|---|---|
| (nM) | Moy | ET | (nM) | Moy | ET |
| 27 | 92 | 2 | 27 | 104 | 4 |
| 82 | 95 | 2 | 82 | 106 | 4 |
| 247 | 93 | 2 | 247 | 105 | 2 |
| 741 | 92 | 5 | 741 | 105 | 1 |
| 2222 | 94 | 5 | 2222 | 105 | 1 |
| 6667 | 93 | 5 | 6667 | 106 | 1 |
| 20000 | 97 | 3 | 20000 | 109 | 5 |

Après traitement avec le métabolite actif du remdesivir, les résultats sont similaires entre les réplicats biologiques avec une viabilité cellulaire moyenne allant de 92% (27 nM, N=1, tableau de gauche ci-dessus) à 109% (20 000 nM, N=2, tableau de droite).

Lors du N=1, la viabilité cellulaire après traitements aux TOTUMs 2, 3 et 4, pour les sept concentrations allant de 14 ng/mL à 10 000 ng/mL étaient similaires à celles obtenues après traitement au métabolite actif du remdesivir.

| cc (ng/mL) | TOTUM 2 Moy | TOTUM 2 ET | TOTUM 3 Moy | TOTUM 3 ET | TOTUM 4 Moy | TOTUM 4 ET |
|---|---|---|---|---|---|---|
| 14 | 91 | 1 | 96 | 4 | 97 | 2 |
| 41 | 89 | 2 | 94 | 6 | 93 | 0 |
| 123 | 89 | 2 | 95 | 3 | 96 | 3 |
| 370 | 88 | 1 | 92 | 4 | 98 | 3 |
| 1111 | 89 | 1 | 93 | 2 | 96 | 2 |
| 3333 | 89 | 0 | 93 | 2 | 97 | 1 |
| 10000 | 90 | 1 | 91 | 1 | 111 | 1 |

Des résultats similaires ont été obtenus pour les TOTUMs 2 et 4 lors du N=2 pour les concentrations testées allant de 137 ng/mL à 100 000 ng/mL.

| cc (ng/mL) | TOTUM 3 | |
| --- | --- | --- |
| | Moy | ET |
| 137 | 97 | 2 |
| 412 | 97 | 2 |
| 1 235 | 99 | 4 |
| 3 704 | 98 | 6 |
| 11 111 | 95 | 9 |
| 33 333 | 32 | 3 |
| 100 000 | 1 | 0 |

| cc (ng/mL) | TOTUM 2 | | TOTUM 4 | |
| --- | --- | --- | --- | --- |
| | Moy | ET | Moy | ET |
| 27 | 100 | 1 | 95 | 1 |
| 82 | 100 | 1 | 96 | 2 |
| 247 | 101 | 1 | 97 | 1 |
| 741 | 100 | 1 | 97 | 2 |
| 2 222 | 100 | 1 | 98 | 1 |
| 6 667 | 100 | 1 | 103 | 2 |
| 20 000 | 100 | 1 | 109 | 4 |

Par ailleurs, pour le TOTUM 3, aux concentrations de 33 333 ng/mL et 100 000 ng/mL, une cytotoxicité est présente.

Résultats : CAS 1 - Effet antiviral des composés

Ces tests ont pour objectif l'évaluation de l'effet antiviral de composés qui doivent être analysés, autrement dit, les cellules sont traitées par le composé avant d'être infectées.

Tout d'abord, sur la lignée cellulaire Calu-3, la charge virale a été évaluée par quantification de l'ARN viral en ciblant le gène ORF1ab. Le contrôle cellule infectée est la référence à 100%.

Après traitement avec le métabolite actif du Remdésivir, les résultats sont similaires entre les réplicats biologiques. La charge virale est décroissante lorsque la concentration en composé augmente, avec :

[0208] - En N=1, un pourcentage par rapport aux cellules infectées et non traitées allant de 84% pour 27 nM de remdesivir à 0% (charge virale indétectable) pour 2 222 nM, 6 667 nM et 20 000 nM,

| (nM) | Métabolite actif du remdesivir | |
| --- | --- | --- |
| | Moy | ET |
| 27 | 84 | 80 |
| 82 | 107 | 4 |
| 247 | 93 | 43 |
| 741 | 22 | 6 |
| 2222 | 0 | 0 |
| 6667 | 0 | 0 |
| 20000 | 0 | 0 |

[0209] - En N=2, un pourcentage par rapport aux cellules infectées et non traitées allant de 92% pour 55 nM de remdesivir à 0% (charge virale indétectable) pour 4 444 nM, 13 333 nM et 40 000 nM.

| (nM) | Métabollite actif du remdesivir | |
| --- | --- | --- |
| | Moy | ET |
| 55 | 92 | 6 |
| 165 | 85 | 12 |
| 494 | 62 | 6 |
| 1481 | 6 | 4 |
| 4444 | 0 | 0 |

(suite)

| (nM) | Métabollite actif du remdesivir | |
|---|---|---|
| | Moy | ET |
| 13333 | 0 | 0 |
| 40000 | 0 | 0 |

Pour les composés testés, lors du N=1, la charge virale allait de 67% (TOTUM 3 à 370 ng/mL) à 280% (TOTUM 2 à 10 000 ng/mL).

| (ng/mL) | TOTUM 2 | | TOTUM 3 | | TOTUM 4 | |
|---|---|---|---|---|---|---|
| | Moy | ET | Moy | ET | Moy | ET |
| 14 | 83 | 6 | 146 | 89 | 83 | 38 |
| 41 | 85 | 18 | 88 | 16 | 80 | 15 |
| 123 | 95 | 6 | 97 | 37 | 79 | 23 |
| 370 | 75 | 9 | 67 | 20 | 80 | 8 |
| 1111 | 81 | 20 | 77 | 5 | 74 | 7 |
| 3333 | 151 | 51 | 96 | 21 | 72 | 10 |
| 10000 | 280 | 62 | 151 | 23 | 122 | 19 |

Lors du N=2, des résultats similaires au N=1 ont été observés pour le TOTUM 2, pour les sept concentrations. Pour le TOTUM 4 à 6 667 ng/mL et 20 000 ng/mL une charge virale de 59% et 49% a été observée.

| (ng/mL) | TOTUM 2 | | TOTUM 4 | |
|---|---|---|---|---|
| | Moy | ET | Moy | ET |
| 27 | 90 | 10 | 83 | 3 |
| 82 | 102 | 31 | 91 | 8 |
| 247 | 89 | 4 | 80 | 22 |
| 741 | 97 | 14 | 85 | 12 |
| 2222 | 111 | 24 | 67 | 10 |
| 6667 | 105 | 5 | 59 | 7 |
| 20000 | 96 | 16 | 49 | 12 |

Par ailleurs, pour le TOTUM 3 à 100 000 ng/mL une charge virale indétectable (0%) a été observée.

| (ng/mL) | TOTUM 3 | |
|---|---|---|
| | Moy | ET |
| 137 | 114 | 28 |
| 412 | 72 | 26 |
| 1235 | 66 | 17 |
| 3704 | 67 | 14 |
| 11111 | 54 | 21 |
| 33333 | 113 | 43 |
| 100000 | 0 | 0 |

Un dosage de trois cytokines (IL6, IP10 et MCP1) a été effectué sur les surnageants de culture cellulaire (en ng/mL) sur la lignée de cellules pulmonaires Calu 3 inoculée avec le SARS-CoV-2. Les cellules ont été traitées avec les produits testés pendant 24 heures puis inoculées avec la souche virale pendant 72 heures. Les concentrations en composés sont indiqués en nM pour le métabolite actif du remdesivir et en ng/mL pour les TOTUMs testés.

L'IL6 est une cytokine pro-inflammatoire, exprimée à un taux basal de 300pg/mL. En cas d'infection son taux est fortement augmenté de l'ordre de 2000pg/mL. La chimiokine IP10 est impliquée dans les processus inflammatoires, indétectable en taux basal. En cas d'infection son taux est fortement augmenté de l'ordre de 400pg/ml

La cytokine MCP1 n'a pas pu être détectée dans les études effectuées. Les cytokines ont une expression transitoire ; par conséquent, au moment où le dosage est réalisé, la cytokine a déjà pu être exprimée ou le sera ultérieurement, étant donné qu'un seul point de lecture est effectué, respectivement à 48h et 72h post infection pour les modèles Vero et Calu, et non une cinétique.

Pour IL6 (tableau ci-dessous à gauche pour le premier réplicat N=1 et à droite pour le deuxième réplicat N=2), un effet dose-réponse a été observé pour le métabolite actif du remdesivir comme attendu, avec des concentrations en cytokines décroissante lorsque les concentrations en composé augmentaient.

| (nM) | Métabolite actif du remdesivir | | (nM) | Métabolite actif du remdesivir | |
| --- | --- | --- | --- | --- | --- |
| | Moy | ET | | Moy | ET |
| 27 | 928 | 212.7 | 55 | 680 | 106.6 |
| 82 | 771 | 60.6 | 165 | 631 | 62.0 |
| 247 | 651 | 35.2 | 494 | 508 | 21.0 |
| 741 | 586 | 280.9 | 1481 | 301 | 23.0 |
| 2222 | 255 | 26.1 | 4444 | 221 | 4.8 |
| 6667 | 273 | NA | 13333 | 172 | 15.1 |
| 20000 | 308 | 49.1 | 40000 | 245 | 57.1 |

Pour la chimiokine IP10 (tableau ci-dessous à gauche pour réplicat N=1 et à droite pour réplicat N=2) un effet dose-réponse est également observé :

| (nM) | Métabolite actif du remdesivir | | (nM) | Métabolite actif du remdesivir | |
| --- | --- | --- | --- | --- | --- |
| | Moy | ET | | Moy | ET |
| 27 | 291 | 94 | 55 | 462 | 26 |
| 82 | 233 | 10 | 165 | 422 | 96 |
| 247 | 205 | 2 | 494 | 382 | 17 |
| 741 | 162 | 106 | 1481 | 62 | 61 |
| 2222 | NA | NA | 4444 | NA | NA |
| 6667 | NA | NA | 13333 | NA | NA |
| 20000 | NA | NA | 40000 | NA | NA |

Il est particulièrement intéressant de noter que des résultats similaires, à savoir un effet dose-réponse, ont été observés pour les TOTUMs 3 et 4 pour le N=1 lors du dosage de l'IL6.

| (ng/mL) | TOTUM3 | | (ng/mL) | TOTUM 4 | |
| --- | --- | --- | --- | --- | --- |
| | Moy | ET | | Moy | ET |
| 14 | 1696 | 762.1 | 14 | 886 | 92 |
| 41 | 1097 | 543.4 | 41 | 710 | 44 |
| 123 | 882 | 324.5 | 123 | 788 | 13 |
| 370 | 828 | 363.6 | 370 | 674 | 150 |
| 1111 | 655 | 231.7 | 1111 | 654 | 95 |
| 3333 | 650 | 220.0 | 3333 | 468 | 26 |
| 10000 | 478 | 51.1 | 10000 | 767 | 162 |

Il en est de même pour le N=2.

| | TOTUM 3 | | | TOTUM 4 | |
|---|---|---|---|---|---|
| (ng/mL) | Moy | ET | (ng/mL) | Moy | ET |
| 137 | 688 | 189.9 | 27 | 1721 | 1059 |
| 412 | 516 | 222.7 | 82 | 206 | 96 |
| 1235 | 463 | 73.1 | 247 | 247 | 95 |
| 3704 | 340 | 141.7 | 741 | 232 | 82 |
| 11111 | 174 | 77.4 | 2222 | 183 | 44 |
| 33333 | 42 | 32.9 | 6667 | NA | NA |
| 100000 | 117 | 20.1 | 20000 | 118 | 72 |

Un effet dose réponse est également remarquable lors des tests avec les TOTUMs 3 et 4 pour le N=1 pour le dosage de l'IP10, pour le réplicat N=1 :

| | TOTUM3 | | | TOTUM 4 | |
|---|---|---|---|---|---|
| (ng/mL) | Moy | ET | (ng/mL) | Moy | ET |
| 14 | 511 | 241 | 14 | 231 | 36 |
| 41 | 325 | 190 | 41 | 166 | 12 |
| 123 | 225 | 101 | 123 | 180 | 6 |
| 370 | 211 | 129 | 370 | 173 | 73 |
| 1111 | 182 | 89 | 1111 | 167 | 38 |
| 3333 | 179 | 74 | 3333 | 103 | 1 |
| 10000 | 91 | 6 | 10000 | 178 | 37 |

Il en est de même pour le N=2 :

| | TOTUM 3 | | | TOTUM 4 | |
|---|---|---|---|---|---|
| (ng/mL) | Moy | ET | (ng/mL) | Moy | ET |
| 137 | 363 | 99 | 27 | 470 | 208 |
| 412 | 273 | 110 | 82 | 340 | 43 |
| 1235 | 215 | 85 | 247 | 350 | 61 |
| 3704 | 149 | 79 | 741 | 406 | 59 |
| 11111 | NA | NA | 2222 | 360 | 50 |
| 33333 | NA | NA | 6667 | 165 | 82 |
| 100000 | NA | NA | 20000 | 117 | 10 |

Aucun effet dose-réponse n'est observé pour le TOTUM 2 que ce soit dans le cadre du dosage de l'IL6 ou dans le cadre du dosage de IP10 (résultats non montrés).

Sur la lignée cellulaire VeroE6-TMPRSS2, la charge virale a été évaluée par quantification de l'ARN viral en ciblant le gène ORF1ab.

Après traitement au métabolite actif du remdesivir, les résultats sont similaires entre les réplicats biologiques. La charge virale est décroissante lorsque la concentration en composé augmente, avec :

- En N=1, un pourcentage par rapport aux cellules infectées et non traitées allant de 101% pour 27 nM de remdesivir à 0% (charge virale indétectable) pour 6 667 nM et 20 000 nM,

|  | Métabolite actif du remdesivir | |
|---|---|---|
| (nM) | Moy | ET |
| 27 | 101 | 15 |
| 82 | 122 | 24 |
| 247 | 120 | 14 |
| 741 | 148 | 14 |
| 2222 | 67 | 27 |
| 6667 | 0 | 0 |
| 20000 | 0 | 0 |

- En N=2, un pourcentage par rapport aux cellules infectées et non traitées allant de 70% pour 27 nM de remdesivir à 0% (charge virale indétectable) pour 6 667 nM et 20 000 nM :

|  | Métabolite actif du remdesivir | |
|---|---|---|
| (nM) | Moy | ET |
| 27 | 70 | 20 |
| 82 | 114 | 44 |
| 247 | 105 | 21 |
| 741 | 144 | 16 |
| 2222 | 7 | 3 |
| 6667 | 0 | 0 |
| 20000 | 0 | 0 |

Lors du N=1, la charge virale par rapport aux cellules infectées et non traitées après traitements aux TOTUMs 2, 3 et 4, pour les sept concentrations allant de 14 ng/mL à 10 000 ng/mL était similaire ou supérieure à celle obtenue après traitement à la dose la plus faible de métabolite actif du remdesivir ; l'activité antivirale ne semble pas être diminuée.

Ces résultats ne sont pas repris ici.

Lors du N=2, des résultats similaires au N=1 ont été observés pour le TOTUM 2 pour les sept concentrations allant de 137 ng/mL à 100 000 ng/mL (pas d'induction de diminution de la charge virale, donc pas d'activité antivirale du TOTUM 2).

Par ailleurs :

- Après traitement au TOTUM 3 aux concentrations de 33 333 ng/mL et 100 000 ng/mL des charges virales de 9% et 0%, respectivement, ont été observées, autrement dit indétectables :

| cc (ng/mL) | TOTUM 3 | |
|---|---|---|
|  | Moy | ET |
| 137 | 129 | 13 |
| 412 | 105 | 14 |
| 1235 | 120 | 18 |
| 3704 | 102 | 17 |
| 11111 | 71 | 10 |
| 33333 | 9 | 7 |
| 100000 | 0 | 0 |

- Après traitement au TOTUM 4 à 6667 ng/mL, une charge virale de 78% par rapport aux cellules infectées non traitées est observée et, pour le traitement avec la concentration de 20 000 ng/mL, une charge virale diminuée à 40% a été observée :

| cc (ng/mL) | TOTUM 4 | |
| --- | --- | --- |
| | Moy | ET |
| 27 | 97 | 15 |
| 82 | 53 | 32 |
| 247 | 96 | 11 |
| 741 | 94 | 7 |
| 2222 | 95 | 12 |
| 6667 | 78 | 8 |
| 20000 | 40 | 5 |

Le TOTUM 3 induit une diminution importante de la charge virale, voire indétectable, aux concentrations de 33 333 ng/mL et 100 000 ng/mL.

Le TOTUM 4 induit une diminution substantielle de la charge virale à la concentration de 20 000 ng/mL.

Résultats : CAS 2 - Effet virucide des composés

Ces tests ont pour objectif l'évaluation de l'effet virucide de composés qui doivent être analysés, autrement dit le virus a été incubé avec les composés pendant 30 minutes avant que les cellules soient mises en culture avec les inoculums prétraités.

Tout d'abord, sur la lignée cellulaire Calu-3, la charge virale a été évaluée par quantification de l'ARN viral en ciblant le gène ORF1ab.

Après traitement avec le métabolite actif du remdesivir, les résultats sont similaires entre les réplicats biologiques. La charge virale est décroissante lorsque la concentration en composé augmente, avec :

- Au N=1, un pourcentage par rapport aux cellules infectées et non traitées allant de 113% pour 27 nM de remdesivir à 0% pour 6 667 nM et 20 000 nM :

| (nM) | Métabolite actif du remdesivir | |
| --- | --- | --- |
| | May | ET |
| 27 | 113 | 32 |
| 82 | 93 | 36 |
| 247 | 82 | 22 |
| 741 | 22 | 5 |
| 2222 | 1 | 1 |
| 6667 | 0 | 0 |
| 20000 | 0 | 0 |

- Au N=2, un pourcentage par rapport aux cellules infectées et non traitées allant de 109% pour 27 nM de remdesivir à 0% pour 2222 nM, 6667 nM et 20 000 nM :

| (nM) | Métabolite actif du remdesivir | |
| --- | --- | --- |
| | Moy | ET |
| 27 | 109 | 68 |
| 82 | 66 | 26 |
| 247 | 56 | 52 |
| 741 | 10 | 4 |
| 2222 | 0 | 0 |
| 6667 | 0 | 0 |
| 20000 | 0 | 0 |

Lors du N=1, la charge virale par rapport aux cellules infectées et non traitées après traitement aux TOTUMs 2, 3 et 4, pour les sept concentrations allant de 14 ng/mL à 10 000 ng/mL étaient similaire ou supérieure à celle obtenue après traitement à la dose la plus faible de métabolite actif du remdesivir (résultats non montrés).

Lors du N=2, des résultats similaires au N=1 ont été observées pour le TOTUM 2 pour les sept concentrations allant de 27 ng/mL à 20 000 ng/mL et de 14 ng/mL à 10 000 ng/mL.

Par ailleurs :

- Après traitement au TOTUM 3 à la concentration de 100 000 ng/mL une charge virale de 0% (charge virale indétectable) a été observée ;

Après traitement au TOTUM 4 aux concentrations de 2 222 ng/mL et 6 667 ng/mL des charges virales de 46% et 43%, respectivement, ont été observées :

| (ng/mL) | TOTUM 4 | |
|---|---|---|
| | Moy | ET |
| 27 | 84 | 13 |
| 82 | 62 | 14 |
| 247 | 68 | 13 |
| 741 | 76 | 14 |
| 2222 | 46 | 7 |
| 6667 | 43 | 6 |
| 20000 | 90 | 8 |

Un dosage de trois cytokines a été effectué pour IL6 et IP10 sur des échantillons de surnageant de culture cellulaire.

Pour IL6, un effet dose-réponse a été observé pour le métabolite actif du remdesivir avec des concentrations en cytokines décroissante lorsque les concentrations en composé augmentaient, pour le réplicat N=1 (tableau ci-dessous à gauche) et pour le réplicat N=2 (tableau ci-dessous à droite).

| (nM) | Métabolite actif du remdesivir | | (nM) | Métabolite actif du remdesivir | |
|---|---|---|---|---|---|
| | Moy | ET | | May | ET |
| 27 | 1617 | 232.5 | 55 | 738 | 25.5 |
| 82 | 1576 | 5.7 | 165 | 657 | 46.9 |
| 247 | 1362 | 180.3 | 494 | 670 | 72.5 |
| 741 | 966 | 33.8 | 1481 | 293 | 13.1 |
| 2222 | 169 | 6.2 | 4444 | 186 | 85.2 |
| 6667 | 154 | 88.2 | 13333 | 224 | 4.4 |
| 20000 | 203 | 55.3 | 40000 | 286 | 13.1 |

Dans le cadre du dosage de l'IL6, des résultats similaires ont été observés pour les TOTUMs 3 et 4 pour le N=1 :

| (ng/mL) | TOTUM 3 | | (ng/mL) | TOTUM 4 | |
|---|---|---|---|---|---|
| | Moy | ET | | Moy | ET |
| 14 | 1917 | 319.6 | 14 | 1269 | 238 |
| 41 | 1535 | 296.9 | 41 | 1369 | 63 |
| 123 | 1643 | 619.3 | 123 | 1041 | 275 |
| 370 | 1670 | 645.1 | 370 | 1082 | 185 |
| 1111 | 1643 | 658.5 | 1111 | 1157 | 65 |
| 3333 | 1339 | 376.6 | 3333 | 1041 | 126 |
| 10000 | 625 | 12.3 | 10000 | 920 | 69 |

Il en est de même pour le réplicat N=2 :

|  | TOTUM3 | | | TOTUM 4 | |
|---|---|---|---|---|---|
| (ng/mL) | Moy | ET | (ng/mL) | Moy | ET |
| 137 | 867 | 163.0 | 27 | 581 | 125 |
| 412 | 803 | 305.2 | 82 | 544 | 37 |
| 1235 | 676 | 137.0 | 247 | 684 | 119 |
| 3704 | 523 | 118.0 | 741 | 578 | 68 |
| 11111 | 204 | 38.0 | 2222 | 475 | 11 |
| 33333 | 35 | 23.3 | 6667 | 297 | 31 |
| 100000 | 162 | 54.0 | 20000 | 227 | 54 |

Pour l'IP10 un effet dose-réponse est également à noter lorsque le virus est incubé avec du remdesivir pour le réplicat N=1 (tableau ci-dessous à gauche) et pour le réplicat N=2 (tableau ci-dessous à droite).

|  | Métabolite actif du remdesivir | |  | Métabolite actif du remdesivir | |
|---|---|---|---|---|---|
| (nM) | Moy | ET | (nM) | Moy | ET |
| 27 | 410 | 42 | 55 | 123 | 28 |
| 82 | 405 | 11 | 165 | 129 | 15 |
| 247 | 309 | 2 | 494 | 133 | 38 |
| 741 | 276 | 11 | 1481 | 48 | 6 |
| 2222 | 21 | 14 | 4444 | 6 | 1 |
| 6667 | 27 | 34 | 13333 | 3 | 4 |
| 20000 | 9 | 5 | 40000 | 17 | 6 |

Des résultats similaires, à savoir un effet dose-réponse, ont été observées pour les TOTUMs 3 et 4 pour le N=1 lors du dosage de l'IP10 :

|  | TOTUM3 | | | TOTUM 4 | |
|---|---|---|---|---|---|
| (ng/mL) | Moy | ET | (ng/mL) | Moy | ET |
| 14 | 555 | 82 | 14 | 393 | 98 |
| 41 | 418 | 52 | 41 | 435 | 50 |
| 123 | 437 | 82 | 123 | 338 | 74 |
| 370 | 436 | 173 | 370 | 344 | 80 |
| 1111 | 385 | 134 | 1111 | 344 | 24 |
| 3333 | 327 | 91 | 3333 | 285 | 18 |
| 10000 | 95 | 28 | 10000 | 168 | 24 |

Il en est de même pour le réplicat N=2 :

| | TOTUM3 | | | TOTUM 4 | |
|---|---|---|---|---|---|
| (ng/mL) | Moy | ET | (ng/mL) | Moy | ET |
| 137 | 116 | 36 | 27 | 110 | 31 |
| 412 | 139 | 39 | 82 | 126 | 4 |
| 1235 | 112 | 40 | 247 | 163 | 10 |
| 3704 | 79 | 5 | 741 | 153 | 7 |
| 11111 | 29 | 5 | 2222 | 108 | 2 |
| 33333 | 8 | 5 | 6667 | 46 | 10 |
| 100000 | 5 | 5 | 20000 | 20 | 2 |

Sur la lignée cellulaire VeroE6-TMPRSS2 à présent, la charge virale a virale a été évaluée par quantification de l'ARN viral en ciblant le gène ORF1ab.

Après traitement avec le métabolite actif du remdesivir, les résultats sont similaires entre les réplicats biologiques. La charge virale est décroissante lorsque la concentration en composé augmente, avec :

- En N=1, un pourcentage par rapport aux cellules non infectées et non traitées allant de 97% pour 27 nM de remdesivir à 0% et 1% (charge virale indétectable) pour 6 667 nM et 20 000 nM, respectivement :

| | Métabolite actif du remdesivir | |
|---|---|---|
| (nM) | Moy | ET |
| 27 | 97 | 12 |
| 82 | 122 | 7 |
| 247 | 109 | 9 |
| 741 | 114 | 9 |
| 2222 | 54 | 1 |
| 6667 | 0 | 0 |
| 20000 | 1 | 1 |

- En N=2, un pourcentage par rapport aux cellules non infectées et non traitées allant de 100% pour 27 nM de remdesivir à 0% (charge virale indétectable) pour 2 222 nM, 6 667 nM et 20 000 nM :

| | Métabolite actif du remdesivir | |
|---|---|---|
| (nM) | Moy | ET |
| 27 | 100 | 14 |
| 82 | 126 | 15 |
| 247 | 113 | 11 |
| 741 | 73 | 3 |
| 2222 | 0 | 0 |
| 6667 | 0 | 0 |
| 20000 | 0 | 0 |

Lors du N=1, la charge virale par rapport aux cellules infectées et non traitées après traitements aux TOTUMs 2, 3 et 4, pour les sept concentrations allant de 14 ng/mL à 10 000 ng/mL était similaire ou supérieure à celle obtenue après traitement à la dose la plus faible de métabolite actif du remdesivir (résultats non montrés, a priori pas d'effet virucide).

Lors du N=2, des résultats similaires au N=1 ont été observées pour le TOTUM 2 pour les sept concentrations allant de 27 ng/mL à 20 000 ng/mL (résultats non montrés, pas d'effet virucide du TOTUM 2).

Par ailleurs :

- Après traitement avec le TOTUM 3 à la concentration de 33333 ng/mL, une charge virale de 16 % est observée ; à la concentration de 100 000 ng/mL, une charge virale indétectable (0%) a été observée :

| cc (ng/mL) | TOTUM 3 | |
|---|---|---|
| | Moy | ET |
| 137 | 84 | 5 |
| 412 | 93 | 15 |
| 1235 | 106 | 6 |
| 3704 | 90 | 20 |
| 11111 | 77 | 8 |
| 33333 | 16 | 13 |
| 100000 | 0 | 0 |

Après traitement au TOTUM 4 à la concentration de 20 000 ng/mL une charge virale de 47% a été observée :

| cc (ng/mL) | TOTUM 2 | | TOTUM 4 | |
|---|---|---|---|---|
| | Moy | ET | Moy | ET |
| 27 | 103 | 17 | 106 | 10 |
| 82 | 115 | 15 | 117 | 30 |
| 247 | 106 | 11 | 111 | 11 |
| 741 | 128 | 11 | 108 | 17 |
| 2222 | 139 | 17 | 115 | 10 |
| 6667 | 148 | 27 | 90 | 0 |
| 20000 | 143 | 14 | 47 | 24 |

Conclusions

Les résultats qui ont été obtenus permettent de démontrer les éléments suivants :
D'une part, le composé remdesivir qui a été testé *in vitro* en tant que métabolite actif de référence contre le virus du SARS-CoV-2, montre bien une activité antivirale et virucide à l'encontre dudit virus, sans toxicité apparente sur les cellules. La charge virale est décroissante lorsque la concentration en remdesivir augmente pour le CAS 1 et le CAS 2. En outre, pour les cytokines IL6 et IP10, un effet dose réponse est observé pour le métabolite actif du remdesivir, avec des concentrations décroissantes en cytokines lorsque les concentrations dudit métabolite augmentent, comme attendu. Ces résultats permettent de valider les protocoles de tests utilisés.

Cela étant, le remdesivir, bien qu'ayant une activité intéressante *in vitro* sur le virus du SARS-CoV-2, présente en parallèle des effets néphrotoxiques notoires pouvant s'avérer néfastes pour un patient atteint par la Covid.

L'échantillon référencé TOTUM 2 a été obtenu à partir de pulpe de banane sèches. Les résultats obtenus lors des tests effectués démontrent qu'un tel extrait ne présente aucun effet antiviral ou virucide. En outre, aucun effet dose réponse sur la libération de cytokines n'a pu être constaté pour cet échantillon.

Là encore, ces résultats de tests, attendus négatifs, confortent le protocole qui a été mis en œuvre.

En ce qui concerne l'échantillon nommé TOTUM 3, celui-ci est obtenu à partir d'une teinture mère de *Neurolaena lobata* concentrée, tandis que l'échantillon référencé sous le nom de TOTUM 4 correspond à une dilution de la teinture mère qui a permis l'obtention également dudit TOTUM 3, comme il ressort de la description détaillée du protocole d'obtention de ces TOTUMs 3 et 4 décrit ci-dessus.

Les résultats obtenus avec le TOTUM 3 et détaillés ci-dessus démontrent, d'une part, que pour les concentrations en composé allant de 14 à 10000 ng/mL, aucune toxicité n'est à relever sur les cellules Calu-3 et VeroE6.

Par contre, une cytotoxicité est à noter pour une concentration en composé de 100 000ng/mL sur les cellules de la lignée Calu-3. Il en est de même pour les concentrations en composé de 33 333ng/mL et de 100 000 ng/mL sur les cellules de la lignée VeroE6, où une cytotoxicité est présente.

En parallèle, pour le TOTUM 3, à une concentration de 100 000 ng/mL, une charge virale indétectable est observée pour la lignée cellulaire Calu-3. Cela étant, cette concentration a été démontrée comme présentant des effets cytotoxiques. Pour la lignée cellulaire VeroE6, des charges virales indétectables ont été observées pour des concentrations en composés de 33 333 ng/mL et de 100 000 ng/mL. Pour autant, là encore une cytotoxicité est présente.

Il semble que la concentration en composé actif dans cet échantillon soit trop importante et entraine une cytotoxicité sur les cellules.

Les résultats obtenus avec le TOTUM 4, consistant en une dilution au quart de la teinture mère, par rapport au TOTUM 3 évoqué ci-dessus, sont, quant à eux, particulièrement intéressants.

Pour mémoire, dans le procédé de préparation des échantillons, suivant l'étape de filtration de la teinture mère, à l'étape iv) du protocole d'obtention du TOTUM 3, on prélève 0,75L de filtrat que l'on dilue dans un volume égal à 2,25L d'eau. On obtient alors une solution hydroalcoolique présentant un volume total égal à 3L et correspondant à une dilution à ¼ de la teinture mère de feuilles sèches de *Neurolaena lobata*.

Les résultats obtenus avec ce TOTUM 4 démontrent, d'une part, que celui-ci ne présente aucune cytotoxicité sur les lignées cellulaires modèles, quelle que ce soit la concentration testée, même pour les plus élevées d'entre elles.

En parallèle de cette absence de cytotoxicité, une activité antivirale du TOTUM 4 est démontrée pour les concentrations 6 667 ng/mL et 20 000 ng/mL, pour lesquelles les charges virales observées sont respectivement de 59 et 49% pour la lignée cellulaire Calu-3. Une activité antivirale a également été détectée pour la lignée VeroE6, après traitement au TOTUM 4 à 6667, à 10 000 ng/mL et à 20 000 ng/mL, pour lesquelles des charges virales de 78%, 66% et 40% ont été respectivement observées.

A noter également que, de manière particulièrement intéressante, lors du dosage des cytokines IL6 et IP10 qui ont été effectués pour le CAS 1 et pour le CAS 2, un effet dose-réponse est observée pour le TOTUM 4, avec des concentrations en cytokines décroissantes lorsque les concentrations en composés augmentent.

En conséquence des résultats qui précèdent, et notamment ceux portant sur le dosage des cytokines IL6 et IP10 dans les cellules pulmonaires, il est possible d'affirmer qu'un extrait de teinture mère dilué, notamment selon une dilution au ¼ de la teinture mère, et lyophilisé de feuilles de *Neurolaena lobata,* en solution liquide présentant une concentration comprise entre 6500 et 20 000 ng/mL (masse d'extrait lyophilisé de teinture mère diluée / volume de solution aqueuse), de préférence entre 6 667 et 20 000 ng/mL, présente une activité antivirale et une activité virucide à l'encontre du virus SARS-CoV-2 responsable de la Covid 19, et présente une efficacité dans la lutte contre les formes graves de cette maladie.

En effet, les résultats démontrant un effet dose dépendante du TOTUM 4 sur la libération des cytokines IL-6 et IP10, un tel extrait de teinture mère de *Neurolaena lobata* est particulièrement indiqué dans l'optique d'éviter l'orage cytokinique susceptible de se produire, en particulier, dans les poumons des malades atteints de forme grave de Covid, en réaction à l'infection par le virus.

On entendra ici par « patient atteint d'une forme grave de Covid-19 », un patient hospitalisé pour lutter contre la Covid-19 et placé sous oxygénothérapie.

**Revendications**

1. Procédé de préparation d'un extrait sec d'une teinture mère diluée de feuilles sèches de *Neurolaena lobata,* **caractérisé en ce qu'**il comporte les étapes suivantes :

   i) on prépare un mélange à base de feuilles sèches de *Neurolaena lobata* dans de l'alcool de canne à sucre à 50°, ledit mélange présentant une concentration massique comprise entre 15 et 20 g/L ;
   ii) on laisse ledit mélange à macérer sous agitation pendant environ 21 jours ;
   iii) Après macération, on filtre le mélange sur « cartouches » de porosité 50-75 μm et on obtient un filtrat liquide, correspondant à une teinture mère de feuilles sèches de *Neurolaena lobata,* et un rétentat solide ;
   iv) on dilue ladite teinture mère liquide à ¼ par ajout d'une solution aqueuse et on obtient alors une solution hydroalcoolique ;
   v) on effectue une évaporation de l'alcool contenu dans cette solution par évaporateur rotatif, jusqu'à obtention d'une solution aqueuse ;
   vi) on congèle la solution aqueuse ainsi obtenue, puis on la lyophilise pour obtenir un extrait sec de la teinture mère diluée de feuilles sèches de *Neurolaena lobata.*

2. Procédé de préparation d'un extrait sec à partir d'une teinture mère diluée de feuilles sèches de *Neurolaena lobata* selon la revendication précédente, **caractérisé en ce que** le mélange préparé à l'étape i) présente une concentration massique comprise entre 16 et 17g de feuilles sèches de *Neurolaena lobata* par litre (L) d'alcool de canne à sucre à 50°, et de préférence égale à 16g/L.

3. Procédé de préparation d'un extrait sec à partir d'une teinture mère diluée de feuilles sèches de *Neurolaena lobata* selon la revendication 1 ou la revendication 2, **caractérisé en ce que**, lors de l'étape iv), on dilue ladite teinture mère en mélangeant un volume égal à 0,75L de filtrat et un volume égal à 2,25L d'eau.

4. Procédé de préparation d'une solution liquide d'un extrait sec de teinture mère diluée et lyophilisé de feuilles sèches de *Neurolaena lobata,* obtenu suivant le procédé de l'une quelconque des revendications 1 à 3, **caractérisé en ce que** ladite solution liquide est obtenue en diluant ledit extrait sec dans de l'eau ou dans un solvant aqueux pharmaceutiquement acceptable, ladite solution liquide présentant une concentration comprise entre 6 500 et 20 000 ng d'extrait sec par mL de solvant aqueux, de préférence entre 6 667 et 20 000 ng/mL.

5. Extrait de teinture mère de feuilles sèches de *Neurolaena lobata* diluée, selon une dilution au ¼ de la teinture mère, et lyophilisé, ledit extrait étant en solution et présentant une concentration finale comprise entre 6 500 et 20 000 ng/mL (masse d'extrait sec lyophilisé de teinture mère diluée / volume de solution aqueuse), pour son utilisation dans le traitement d'une infection virale due au virus SARS-CoV-2 responsable de la Covid 19.

6. Extrait de teinture mère de feuilles de *Neurolaena lobata* selon la revendication précédente pour son utilisation dans la diminution de la production de cytokines, notamment IL-6 et IP-10, dans le traitement des formes graves d'une infection virale due au virus SARS-CoV-2 responsable de la Covid 19.

**Patentansprüche**

1. Verfahren zur Herstellung eines Trockenextrakts einer verdünnten Urtinktur aus getrockneten Blättern von *Neurolaena lobata,*

   **dadurch gekennzeichnet, dass** es die folgenden Schritte umfasst:

   i) Zubereiten einer Mischung auf der Basis von getrockneten Blättern von *Neurolaena lobata* in 50°-Zuckerrohralkohol, wobei diese Mischung eine Massenkonzentration zwischen 15 und 20 g/l aufweist;
   ii) Mazerieren dieser Mischung unter Rühren für etwa 21 Tage;
   iii) nach dem Mazerieren, Filtrieren der Mischung über "Kartuschen" mit einer Porosität von 50 - 75 $\mu$m und Erhalten eines flüssigen Filtrats, das einer Urtinktur aus getrockneten Blättern von *Neurolaena lobata* entspricht, sowie ein festes Retentat;
   iv) Verdünnen dieser flüssigen Urtinktur auf ¼ durch Zugabe einer wässrigen Lösung und anschließend Erhalten einer hydroalkoholischen Lösung;
   v) Durchführen einer Verdampfung des in dieser Lösung enthaltenen Alkohols mittels Rotationsverdampfer bis zum Erhalten einer wässrigen Lösung;
   vi) Einfrieren der somit erhaltenen wässrigen Lösung, dann Lyophilisieren von dieser, um einen Trockenextrakt der verdünnten Urtinktur aus getrockneten Blättern von *Neurolaena lobata* zu erhalten.

2. Verfahren zur Herstellung eines Trockenextrakts aus einer verdünnten Urtinktur aus getrockneten Blättern von *Neurolaena lobata* nach dem vorstehenden Anspruch, **dadurch gekennzeichnet, dass** die in Schritt i) hergestellte Mischung eine Massenkonzentration zwischen 16 und 17g getrockneter Blätter von *Neurolaena lobata* pro Liter (l) 50°-Zuckerrohralkohol aufweist, und vorzugsweise gleich 16 g/l ist.

3. Verfahren zur Herstellung eines Trockenextrakts aus einer verdünnten Urtinktur aus getrockneten Blättern von *Neurolaena lobata* nach Anspruch 1 oder Anspruch 2, **dadurch gekennzeichnet, dass** in Schritt iv) diese Urtinktur durch Mischen eines Volumens von 0,75 l Filtrat mit einem Volumen von 2,25 l Wasser verdünnt wird.

4. Verfahren zur Herstellung einer flüssigen Lösung eines Trockenextrakts aus verdünnter und lyophilisierter Urtinktur aus getrockneten Blättern von *Neurolaena lobata,* die entsprechend dem Verfahren nach einem der Ansprüche 1 bis 3 erhalten wird, **dadurch gekennzeichnet, dass** diese flüssige Lösung durch Verdünnen des Trockenextrakts in Wasser oder in einem pharmazeutisch unbedenklichen wässrigen Lösungsmittel erhalten wird, wobei diese flüssige Lösung eine Konzentration zwischen 6.500 und 20.000 ng Trockenextrakt pro ml wässrigem Lösungsmittel, vorzugsweise zwischen 6.667 und 20.000 ng/ml, aufweist.

5. Extrakt aus Urtinktur aus getrockneten Blättern von *Neurolaena lobata,* verdünnt gemäß einer Verdünnung auf ¼ der Urtinktur und lyophilisiert, wobei dieser Extrakt in Lösung vorliegt und eine Endkonzentration zwischen 6.500 und 20.000 ng/ml aufweist (Masse des lyophilisierten Trockenextrakts der verdünnten Urtinktur / Volumen der wässrigen Lösung), für dessen Verwendung zur Behandlung einer Virusinfektion durch das SARS-CoV-2-Virus, das für Covid 19 verantwortlich ist.

6. Extrakt aus Urtinktur aus Blättern von *Neurolaena lobata* nach dem vorstehenden Anspruch für dessen Verwendung bei der Verringerung der Produktion von Zytokinen, insbesondere IL-6 und IP-10, bei der Behandlung schwerer Formen einer Virusinfektion durch das SARS-CoV-2-Virus, das für Covid 19 verantwortlich ist.

**Claims**

1. Method for preparing a dry extract of a diluted mother tincture of dried leaves of *Neurolaena lobata,* **characterized in that** it comprises the following steps:

   i) a mixture of dried leaves of *Neurolaena lobata* in sugar cane alcohol at 50° is prepared, said mixture having a mass concentration of between 15 and 20 g/L;
   ii) said mixture is allowed to macerate under agitation for about 21 days;
   iii) After maceration, the mixture is filtered on "cartridges" of porosity 50-75 $\mu$m and a liquid filtrate is obtained, corresponding to a mother tincture of dried leaves of *Neurolaena lobata,* and a solid retentate;
   iv) said liquid mother tincture is diluted to ¼ by adding an aqueous solution, and an aqueous-alcoholic solution is thus obtained;
   v) the alcohol contained in this solution is evaporated by a rotary evaporator, until an aqueous solution is obtained;
   vi) the aqueous solution thus obtained is frozen and then freeze-dried to obtain a dry extract of the diluted mother tincture of dried leaves of *Neurolaena lobata.*

2. Method for preparing a dry extract from a diluted mother tincture of dried leaves of *Neurolaena lobata* according to the preceding claim, **characterized in that** the mixture prepared in step i) has a mass concentration comprised between 16 and 17 g of dried leaves of *Neurolaena lobata* per liter (L) of sugar cane alcohol at 50°, and preferably equal to 16 g/L.

3. Method for preparing a dry extract from a diluted mother tincture of dried leaves of *Neurolaena lobata* according to claim 1 or claim 2, **characterized in that,** during step iv), said mother tincture is diluted by mixing a volume equal to 0.75 L of filtrate and a volume equal to 2.25 L of water.

4. Method for preparing a liquid solution from a dry extract of freeze-dried diluted mother tincture of dried leaves of *Neurolaena lobata,* obtained according to the method of any of claims 1 to 3, **characterized in that** said liquid solution is obtained by diluting said dry extract in water or in a pharmaceutically acceptable aqueous solvent, said liquid solution having a concentration of between 6,500 and 20,000 ng of dry extract per mL of aqueous solvent, preferably between 6,667 and 20,000 ng/mL.

5. Diluted mother tincture extract of dried leaves of *Neurolaena lobata,* diluted to ¼ of the mother tincture, and freeze-dried, said extract being in a solution and having a final concentration of between 6,500 and 20,000 ng/mL (mass of freeze-dried dry extract of diluted mother tincture / volume of aqueous solution), for its use in the treatment of a viral infection due to the SARS-CoV-2 virus responsible for Covid-19.

6. Mother tincture extract of *Neurolaena lobata* leaves according to the preceding claim, for its use in the decrease in the production of cytokines, in particular IL-6 and IP-10, in the treatment of the serious forms of a viral infection due to the SARS-CoV-2 virus responsible for Covid-19.

[Fig.1]

[Fig.2]

[Fig.3]

**Courbe d'évolution de la DO à 600 nm en fonction du temps de mise en contact de la DHODH et de son substrat avec un échantillon à 0,01 µg/mL**

---H1 ——H2 ···· Brequinar

[Fig.4]

**Courbe d'évolution de la DO à 600 nm en fonction du temps de mise en contact de la DHODH et de son substrat avec un échantillon à 0,1 µg/mL**

-·-H1 ——H2 ····Brequinar

[Fig.5]

**Courbe d'évolution de la DO à 600 nm en fonction du temps de mise en contact de la DHODH et de son substrat avec un échantillon à 0,1 µg/mL**

Temps en secondes

— ◆ —H1    ——H2    •••••Brequinar

[Fig.6]

**Courbe du pourcentage d''inhibition de la DHODH, observé entre 0 et 275 secondes de mise en contact, en fonction de la concentration des échantillons H1 et H2**

Concentration d'échantillon en µg/mL

— ◆ —H1    ——H2

[Fig 7]

Courbe du pourcentage d'inhibition de la DHODH, observé entre 0 et 275 secondes de mise en contact, en fonction de la concentration des échantillons H1, H2 ou du brequinar,

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1169036 A

- US 6841561 B

**Littérature non-brevet citée dans la description**

- **GRACIOSO J.S. et al.** *J. Pharm. Pharmacol.*, 1998, vol. 50, 1425-1429
- **GRACIOSO J.S. et al.** *Phytomedecine*, 2000, vol. 7 (4), 283-289

- **C.-T. K. TSENG ; J. TSENG ; L. PERRONE ; M. WORTHY ; V. POPOV ; C. J. PETERS**. Apical entry and release of severe acute respiratory syndrome-associated coronavirus in polarized Calu-3 lung epithelial cells. *J Virol*, August 2005, vol. 79 (15), 9470-9479